# EUROPEAN PATENT APPLICATION

(11) **EP 2 524 701 A2**
(43) Date of publication of application: **21.11.2012**
(21) Application number: 12003909.4
(22) Date of filing: 18.05.2012
(51) Int. Cl.: A61K 39/36, A61K 9/00, A61K 47/02, A61K 47/12, A61P 37/08

(54) **Pharmaceutical composition and method for producing the same**

(30) Priority: 20.05.2011 JP 2011114042; 20.05.2011 JP 2011114045; 20.05.2011 JP 2011114047; 07.05.2012 JP 2012106209
(71) Applicant: Nitto Denko Corporation, Ibaraki-shi, Osaka 567-8680 (JP)
(72) Inventor: Asari, Daisuke, Ibaraki-shi Osaka 567-8680 (JP); Hori, Mitsuhiko, Ibaraki-shi Osaka 567-8680 (JP); Shishido, Takuya, Ibaraki-shi Osaka 567-8680 (JP)
(74) Representative: Müller-Boré & Partner Patentanwälte

(57) **Abstract**

An object of the present invention is to provide a pharmaceutical composition that allows stable storage and delivery of heat-labile allergens. The present invention provides a pharmaceutical composition containing: an allergen; and at least one selected from the group consisting of an organic acid salt, an inorganic acid salt, and a pH adjuster.

## Description

The present invention relates to a pharmaceutical composition that is an agent useful in the treatment or prevention of allergy symptoms. In particular, the present invention relates to a pharmaceutical composition that can stably maintain an allergen and is very user-friendly in terms of storage, handleability, and the like, and a method for preparing such a pharmaceutical composition.

Current treatments for allergic diseases such as pollen allergies are mostly symptomatic treatments with antihistamines, but recent attention has been focused on hyposensitization therapy as definitive therapy for allergic diseases.
The hyposensitization therapy requires long-term administration of a drug (generally, about two to three years), thereby creating a demand for dosage forms that improve the QOL (quality of life) of caregivers and patients.

Current drugs for specific hyposensitization therapies are mostly in the form of injections for subcutaneous injection.
However, specific hyposensitization therapy with subcutaneous injections has disadvantages such as a high risk of anaphylactic shock, a need for injection by a healthcare professional, a need for long-term, frequent hospital visits, pain associated with injection, and storage of the injections under refrigeration.

In contrast, liquid and tablet preparations for sublingual administration which have been recently marketed in Europe and the United States are receiving attention because of their reduced side-effects and user-friendliness.
However, specific hyposensitization therapy by sublingual administration of liquid preparations still has disadvantages such as inaccuracy of the dose and storage of the preparations under refrigeration.
On the other hand, specific hyposensitization therapy by sublingual administration of tablet preparations has disadvantages such as accidental intake, difficulty in controlling the dose, poor portability, and unpleasant sensation in the mouth due to residues.

In addition, in order to develop allergen preparations, stable preservation of the allergens should be secured, that is, the loss of the biological activity of the allergens should be minimized.
Some techniques to formulate allergen preparations have been proposed, for example, in the following Patent Literatures. Patent Literature 1 teaches drying a biological sample containing, as a stabilizer, a glass forming polyol selected from the group consisting of glucose, maltulose, iso-maltulose, lactulose, sucrose, maltose, lactose, sorbitol, iso-maltose, maltitol, lactitol, palatinit, trehalose, raffinose, stachyose, melezitose, and dextran, into a highly viscous liquid for preservation. Patent Literature 2 teaches another technique similar to that of Patent Literature 1, that is, a highly viscous liquid obtained from a biological sample that contains sugars including a first component selected from sucrose and trehalose and a second component selected from the group consisting of mannitol, raffinose, lactitol, sorbitol, and lactobionic acid. Patent Literature 3 teaches a method for stabilizing or solubilizing a protein which includes contacting the protein with a sugar polymer derivative, and specifically teaches, as the sugar polymer derivative, erythrose, threose, ribose, arabinose, xylose, lyxose, allose, altrose, glucose, mannose, gulose, idose, galactose, talose, psicose, fructose, sorbose, tagatose, xylulose, and ribulose. Patent Literature 4 teaches a composition for stabilization of protein agents which contains a surface active substance, a mixture of at least two amino acids, a disaccharide, and ethylenediaminetetraacetic acid, and specifically teaches, as the disaccharide, sucrose, trehalose, and lactose. Patent Literature 5 teaches a stabilizer composition containing at least one amino acid, at least one sugar, and at least one polyamine, and specifically teaches, as the sugar, glucose, lactose, maltol, trehalose, sorbitol, and mannitol. Patent Literature 6 teaches a method for stabilizing a liquid vaccine by adding trehalose.

In addition, other examples of techniques to formulate allergen preparations include methods involving the use of a freeze-drying agent containing a stabilizer or excipient, as disclosed, for example, in the following Patent Literatures.
Patent Literature 7 teaches a pharmaceutical composition containing a Phleum pratense grass pollen allergen in a stable state that is achieved by freeze-drying a solution thereof containing, as stabilizers, mannitol and one of gelatin and starch. Patent Literature 7 states that pH is preferably adjusted prior to solidification of the matrix-containing solution to avoid denaturation of the allergen, precipitation and assure a stable product, and that the pH of the solution is preferably 3.5 to 10, more preferably 4 to 9, and most preferably 6 to 9. Patent Literature 8 teaches a pharmaceutical composition containing a peptide derived from the Cryptomeria japonica pollen allergen in a stable state that is achieved by freeze-drying a solution thereof containing mannitol as a stabilizer and sodium phosphate as a pH adjuster. Patent Literature 9 teaches a pharmaceutical composition containing a recombinant protein of a major Cryptomeria japonica pollen allergen in a stable state that is achieved by freeze-drying a solution thereof containing mannitol as a stabilizer and acetic acid as a pH adjuster. Another example is a pharmaceutical composition containing a recombinant protein of a mite major allergen in a stable state that is achieved by freeze-drying a solution thereof containing macrogol 4000, Polysorbate-80, and sucrose.
These conventional techniques to formulate allergen preparations, however, still encounter difficulties in stable preservation and delivery of allergens due to heat lability of allergens.

### - Patent Literature

Patent Literature 1: JP-T 2006-504801
Patent Literature 2: JP-T 2007-535514
Patent Literature 3: JP-T 2008-501639
Patent Literature 4: JP-T 2006-528137
Patent Literature 5: JP-T 2008-532977
Patent Literature 6: JP-T 2002-540079
Patent Literature 7: JP-T 2006-513269
Patent Literature 8: JP-B 4179422
Patent Literature 9: JP-B 3932272

In view of the above problems, an object of the present invention is to provide a pharmaceutical composition that allows stable preservation and delivery of a heat-labile allergen and a method for preparing such a pharmaceutical composition.

As a result of intensive studies to solve the above problems, the present inventors have found that even a heat-labile allergen can be stably preserved and delivered by adjusting pH with at least one stabilizer selected from the group consisting of an organic acid salt, an inorganic acid salt, and a pH adjuster, thereby completing the present invention.

Specifically, the present invention provides a pharmaceutical composition containing an allergen and at least one selected from the group consisting of an organic acid salt, an inorganic acid salt, and a pH adjuster.
Preferably, the organic acid salt in the pharmaceutical composition of the present invention includes at least one selected from the group consisting of calcium lactate, sodium citrate, calcium citrate, sodium malate, dipotassium glycyrrhizate, disodium glycyrrhizate, calcium gluconate, sodium gluconate, magnesium gluconate, sodium stearyl fumarate, sodium tartrate, potassium sodium tartrate, disodium succinate, sodium acetate, sodium L-aspartate, and sodium L-ascorbate.
Preferably, the inorganic acid salt includes at least one selected from the group consisting of calcium carbonate, (anhydrous) calcium hydrogen phosphate, magnesium carbonate, calcium silicate, magnesium silicate, magnesium aluminometasilicate, synthetic aluminum silicate, sodium hydrogen carbonate, sodium hydrogen phosphate, sodium dihydrogen phosphate, potassium hydrogen carbonate, potassium dihydrogen phosphate, and calcium dihydrogen phosphate.

Preferably, the allergen in the pharmaceutical composition of the present invention is a Cryptomeria japonica pollen allergen protein.
Preferably, the pharmaceutical composition of the present invention further contains gelatin.
Preferably, the pharmaceutical composition of the present invention further contains water.
Preferably, the pH adjuster is capable of adjusting the pharmaceutical composition to a pH of 5.5 to 8.5.
Preferably, the pH adjuster in the pharmaceutical composition of the present invention includes at least one selected from the group consisting of acetic acid, phosphoric acid, boric acid, a mixture of these, sodium hydroxide, and sodium carbonate.

Preferably, the pharmaceutical composition of the present invention does not contain water. Preferably, the pharmaceutical composition of the present invention is a solid preparation, a liquid preparation, or a jelly preparation.
Preferably, the pharmaceutical composition of the present invention is for oral administration.
Preferably, the pharmaceutical composition of the present invention is for hyposensitization therapy.
Preferably, the pharmaceutical composition of the present invention is for administration by subcutaneous injection.

The present invention also provides a method for producing a pharmaceutical composition which includes dissolving or dispersing, in water, an allergen and at least one selected from the group consisting of an organic acid salt, an inorganic acid salt, and a pH adjuster, thereby providing an allergen-containing aqueous solution; and lyophilizing the allergen-containing aqueous solution.
Preferably, in the method for producing a pharmaceutical composition of the present invention, gelatin is further dissolved in the allergen-containing aqueous solution.
Preferably, the allergen-containing aqueous solution has a pH of 5.5 to 8.5.
The following description is offered to illustrate the present invention in detail.

The pharmaceutical composition of the present invention contains an allergen and at least one selected from the group consisting of an organic acid salt, an inorganic acid salt, and a pH adjuster.
In the pharmaceutical composition of the present invention, the organic acid salt and the inorganic acid salt are materials for improving the stability of the allergen.
In the pharmaceutical composition of the present invention, the pH adjuster adjusts the pharmaceutical composition of the present invention to a pH in the specific range so that even a heat-labile allergen can be stably preserved and delivered.

Preferably, the organic acid salt in the pharmaceutical composition of the present invention is, for example, at least one selected from the group consisting of calcium lactate, sodium citrate, calcium citrate, sodium malate, dipotassium glycyrrhizate, disodium glycyrrhizate, calcium gluconate, sodium gluconate, magnesium gluconate, sodium stearyl fumarate, sodium tartrate, potassium sodium tartrate, disodium succinate, sodium acetate, sodium L-aspartate, and sodium L-ascorbate.
In the present invention, the organic acid salt may be, for example, a combination of an organic acid and a base which gives such an organic acid salt as listed above in the pharmaceutical composition of the present invention, and specifically may be a combination of an organic acid and sodium chloride, calcium chloride, magnesium chloride, potassium chloride, or the like.

Preferably, the inorganic acid salt in the pharmaceutical composition of the present invention includes at least one selected from the group consisting of, for example, calcium carbonate, (anhydrous) calcium hydrogen phosphate, magnesium carbonate, calcium silicate, magnesium silicate, magnesium aluminometasilicate, synthetic aluminum silicate, sodium hydrogen carbonate, sodium hydrogen phosphate, sodium dihydrogen phosphate, potassium hydrogen carbonate, potassium dihydrogen phosphate, and calcium dihydrogen phosphate.
In the present invention, the inorganic acid salt may be, for example, a combination of an inorganic acid and a base which gives such an inorganic acid salt as listed above in the pharmaceutical composition, and specifically may be a combination of an inorganic acid and sodium chloride, calcium chloride, magnesium chloride, potassium chloride, or the like.

Since allergens are generally low pH materials, organic acid salts and/or inorganic acid salts that are capable of maintaining a low pH for the allergen are preferable in the present invention. Specifically, preferred examples thereof include organic acid salts and/or inorganic acid salts having a pH of less than 5.5.
More specifically, examples of organic acid salts that are capable of stabilizing the allergen while maintaining a low pH for the allergen include calcium lactate (pH 4.8), dipotassium glycyrrhizate (pH 4.7), sodium tartrate (pH 3.8), sodium ascorbate (pH 5.0), sodium malate (pH 5.4), and sodium stearyl fumarate (pH 4.1). Any of these organic acid salts may be used alone, or two or more of these may be used in combination.
More specifically, examples of inorganic acid salts that are capable of stabilizing the allergen while maintaining a low pH for the allergen include synthetic aluminum silicate (pH 4.6), sodium dihydrogen phosphate (pH 4.4), calcium dihydrogen phosphate (pH 4.1), and potassium dihydrogen phosphate (pH 4.4). Any of these inorganic acid salts may be used alone, or two or more of these may be used in combination.

For example, in the case of that the pharmaceutical composition of the present invention is used as an injection, the amount of the organic acid salt and/or the inorganic acid salt is preferably 0.1 to 5% by weight. If the amount is less than 0.1% by weight, the effect of stabilizing the allergen may be weak; if the amount is more than 5% by weight, the organic acid salt and/or the inorganic acid salt may cause a safety problem, although depending on the properties thereof.
In the case that the pharmaceutical composition of the present invention is used as a liquid preparation for oral administration, the amount of the organic acid salt and/or the inorganic acid salt is preferably 0.1 to 20% by weight. If the amount is less than 0.1% by weight, the effect of stabilizing the allergen may be weak; if the amount is more than 20% by weight, the organic acid salt and/or the inorganic acid salt may cause precipitation of solids, although depending on the properties thereof.
In the case that the pharmaceutical composition of the present invention is used as a solid preparation, the amount of the organic acid salt and/or the inorganic acid salt is preferably 0.1 to 30% by weight. If the amount is less than 0.1% by weight, the effect of stabilizing the allergen may be weak; if the amount is more than 30% by weight, the physical properties of the organic acid salt and/or the inorganic acid salt may have an impact on the solid preparation.

Preferably, the pH adjuster in the pharmaceutical composition of the present invention is one that is capable of adjusting the pharmaceutical composition to a pH in a range described below and has proven to be usable for oral administration. Examples of such pH adjusters include those that have proven to be usable as additives for pharmaceutical compositions, such as adipic acid, aqueous ammonia, hydrochloric acid, sodium carbonate, dilute hydrochloric acid, citric acid hydrate, glycine, glucono-δ-lactone, gluconic acid, sodium dihydrogen phosphate (crystal), succinic acid, acetic acid, ammonium acetate, sodium acetate hydrate, diisopropanolamine, tartaric acid, D-tartaric acid, potassium hydroxide, calcium hydroxide, sodium hydroxide, magnesium hydroxide, sodium hydrogen carbonate, sodium carbonate hydrate, triisopropanolamine, triethanolamine, carbon dioxide, lactic acid, sodium lactate, glacial acetic acid, monosodium fumarate, fumaric acid, sodium propionate, boric acid, ammonium borate, borax, maleic acid, anhydrous citric acid, anhydrous sodium monohydrogen phosphate, anhydrous sodium dihydrogen phosphate, meglumine, methanesulfonic acid, monoethanolamine, sulfuric acid, aluminum potassium sulfate hydrate, DL-malic acid, phosphoric acid, trisodium phosphate, dipotassium phosphate, potassium dihydrogen phosphate, and sodium dihydrogen phosphate. Any of these pH adjusters may be used alone, or two or more of these may be used in combination.

For the production of the pharmaceutical composition of the present invention, the pH adjuster is preferably one that is capable of the pH adjustment even when used in a small amount.
Examples of such pH adjusters include those that have proven to be usable as additives in pharmaceutical compositions, such as adipic acid, aqueous ammonia, hydrochloric acid, sodium carbonate, dilute hydrochloric acid, citric acid hydrate, glycine, glucono-δ-lactone, gluconic acid, sodium dihydrogen phosphate (crystal), succinic acid, acetic acid, ammonium acetate, sodium acetate hydrate, diisopropanolamine, tartaric acid, potassium hydroxide, calcium hydroxide, sodium hydroxide, magnesium hydroxide, sodium hydrogen carbonate, sodium carbonate hydrate, triisopropanolamine, triethanolamine, carbon dioxide, lactic acid, sodium lactate, glacial acetic acid, fumaric acid, monosodium fumarate, sodium propionate, boric acid, ammonium borate, borax, maleic acid, anhydrous citric acid, anhydrous sodium hydrogen phosphate, anhydrous sodium dihydrogen phosphate, meglumine, methanesulfonic acid, monoethanolamine, sulfuric acid, aluminum potassium sulfate hydrate, DL-malic acid, phosphoric acid, trisodium phosphate, dipotassium phosphate, potassium dihydrogen phosphate, and sodium dihydrogen phosphate. Any of these pH adjusters may be used alone, or two or more of these may be used in combination.

Organic acids and organic acid salts that inhibit denaturation and aggregation of proteins are also preferable as the pH adjuster. Examples of such pH adjusters include citric acid hydrate, glycine, glucono-δ-lactone, gluconic acid, succinic acid, acetic acid, sodium acetate hydrate, tartaric acid, lactic acid, glacial acetic acid, fumaric acid, monosodium fumarate, sodium propionate, maleic acid, anhydrous citric acid, and malic acid.
Alternatively, the pH adjuster in the pharmaceutical composition of the present invention is preferably a buffer that has maximum buffering capacity at a pH in a range described below, or a buffer containing an organic acid or organic acid salt that inhibits denaturation and aggregation of proteins.
Specific examples thereof include a citric acid buffer (a mixture of citric acid and sodium citrate), an acetic acid buffer (a mixture of acetic acid and sodium acetate), a citric acid-phosphoric acid buffer (a mixture of citric acid and disodium hydrogen phosphate), and a phosphoric acid buffer (a mixture of sodium dihydrogen phosphate and disodium hydrogen phosphate).
The pH adjuster may be Britton-Robinson buffer, which is a universal buffer, as long as it satisfies the pH range described below.
The Britton-Robinson buffer is not particularly limited, as long as it adjusts the pharmaceutical composition of the present invention to a pH in a range described below. Suitable examples thereof include a mixture containing acetic acid, phosphoric acid, and boric acid at a weight ratio of 1:1:1.
In particular, the pH adjuster in the pharmaceutical composition of the present invention preferably includes one that contains at least one selected from the group consisting of acetic acid, phosphoric acid, boric acid, a mixture of these, sodium hydroxide, and sodium carbonate.

Instead, the pH adjuster may be, for example, a combination of an organic acid and a base which gives such a pH adjuster as listed above in the pharmaceutical composition, and specifically may be a combination of an organic acid and sodium chloride, calcium chloride, magnesium chloride, potassium chloride, or the like.

The amount of the pH adjuster is not particularly limited, but is preferably determined so that the pH of the pharmaceutical composition of the present invention is appropriately controlled in the range described below.

The term "allergen" herein refers to an antigen with which an antibody from an allergy sufferer specifically reacts, and typically refers to a protein.
Specific examples thereof include allergens derived from pollens of trees (golden acacia, red alder, white ash, American beech, birch, box elder, mountain cedar, red cedar, common cottonwood, cypress, American elm, Chinese elm, Japanese Douglas fir, sweet gum, eucalyptus, hackberry, hickory, linden, sugar maple, mesquite, mulberry, oak, olive, pecan tree, pepper tree, pine, common privet, Russian olive, American sycamore, tree of heaven, black walnut, black willow, etc.); allergens derived from pollens of grasses (cotton, Bermuda grass, Kentucky bluegrass, smooth brome, cultivated corn, meadow fescue, Johnson grass, cultivated oats, orchard grass, redtop, perennial rye grass, rice, sweet vernal grass, timothy, careless weed, pigweed, common cocklebur, sorrel dock, goldenrod, kochia, lamb's quarters, marigold, nettle, pigwood, English plantain, giant ragweed, short ragweed, western ragweed, Russian thistle, sagebrush, Scotch broom, sheep sorrel, etc.); allergens derived from insects (silkworm, mite, honeybee, wasp, ant, cockroach, etc.); allergens derived from fungi (Alternaria tenuis, Aspergillus fumigatus, Botrytis cinerea, Candida albicans, Cephalosporium acremonium, Curvularia spicifera, Epicoccum nigrum, Epidermophyton floccosum, Fusarium vasinfectum, Helminthosporium interseminatum, Hormodendrum cladosporioides, Mucor rasemosus, Penicillium notatum, Phoma herbarium, Pullularia pullulans, Rhizopus nigricans, etc.); allergens derived from the skin and hair of animals (dog, cat, bird, etc.); and allergens derived from house dust; and allergens derived from foods. The allergen is not particularly limited, provided that it is an antigen with which an antibody from an allergy sufferer specifically reacts.

Currently, there is a great demand for hyposensitization therapy for cedar pollen allergy from which so many people suffer. Therefore, the allergen in the pharmaceutical composition of the present invention is preferably a cedar (Cryptomeria japonica) pollen allergen protein.
Examples of the cedar pollen allergen protein include those containing, as an active ingredient, at least one selected from the group consisting of antigenic proteins that are extracted from cedar pollens and specifically react with antibodies from allergy sufferers, and proteins having high amino acid sequence identity to these antigenic proteins.

Examples of the antigenic proteins extracted from cedar pollens include proteins in cedar pollens which induce the production of cedar pollen-specific IgE antibodies. These proteins in cedar pollens are composed of major cedar pollen allergen proteins and minor cedar pollen allergen proteins.
Among these cedar pollen extracts from cedar pollens, those to which a large number of sufferers are sensitive are referred to as major cedar pollen allergen proteins, and those to which a small number of sufferers are sensitive are referred to as minor cedar pollen allergen proteins.

The cedar pollen allergen protein may be present in a liquid or a solid. Such a liquid is referred to as a cedar pollen extract. In the case of a liquid cedar pollen extract, the pharmaceutical composition of the present invention may be prepared by combining the extract with the organic acid salt and/or the inorganic acid salt described herein as stabilizer(s), and may be used as an injection or liquid preparation for oral administration as it is prepared, or the pharmaceutical composition may be further combined with a gelling agent and therefore solidified as a solid preparation for oral administration.
Alternatively, the pharmaceutical composition of the present invention may be prepared as a solid preparation for oral administration by mixing the cedar pollen extract with the organic acid and/or the inorganic acid, and processing the mixture by treatment such as lyophilization.

Particularly preferred examples of cedar pollen extracts include those containing major cedar pollen allergen proteins such as Cry j 1 and Cry j 2, and a mixture of these. In the present invention, a cedar pollen extract extracted from cedar pollens which contains a minor cedar pollen allergen protein in addition to Cry j 1 and Cry j 2 is also preferably used as it is, and a diluted product or lyophilized solid product thereof is also preferably used. Specific examples of commercially available pharmaceutical products corresponding to these cedar pollen extracts include standardized allergen extract for subcutaneous injection "Torii" cedar pollen 200 JAU/mL and standardized allergen extract for subcutaneous injection "Torii" cedar pollen 2000 JAU/mL (both from Torii Pharmaceutical Co., Ltd.).
The term "JAU" is an abbreviation for "Japanese Allergy Units", and indicates the potency of a cedar pollen allergen standalized by the major cedar pollen allergen protein Cry j 1.

The amount of the allergen, although depending on the properties of the allergen, is generally 1 × 10⁻¹⁰ to 60% by weight with respect to the total weight of the pharmaceutical composition of the present invention. If the amount is less than 1 × 10⁻¹⁰% by weight, the pharmaceutical composition may not be suitable for hyposensitization therapy; if the amount is more than 60% by weight, a film preparation formed from the pharmaceutical composition of the present invention may have remarkably reduced strength and therefore may have disadvantageously poor shape retainability.

In particular, in the case that the pharmaceutical composition contains a cedar pollen allergen protein as the allergen and also contains the organic acid salt and/or the inorganic acid salt, the amount of the cedar pollen allergen protein, although depending on the properties of the allergen, is preferably 1 × 10⁻¹⁰ to 100% by weight of the amount of the organic acid salt and/or the inorganic acid salt added. If the amount is more than 100% by weight, the organic acid salt and/or the inorganic acid salt may not function as stabilizer(s) enough in terms of storage stability; if the amount is less than 1 × 10⁻¹⁰% by weight, the cedar pollen allergen protein may not provide an optimal clinical effect for practical use.

In the case that the pharmaceutical composition of the present invention contains the pH adjuster, the pH of the pharmaceutical composition is preferably 5.5 to 8.5. If the pH of the pharmaceutical composition of the present invention which contains the pH adjuster is less than 5.5 or more than 8.5, the physicochemical stability of the allergen is remarkably reduced, resulting in a safety problem. It should be noted that the pH of the standardized allergen extracts is specified in the range of 4 to 5. The pH of the pharmaceutical composition can be controlled in the above range with the pH adjuster.

In the case that the pharmaceutical composition of the present invention contains the organic acid salt and/or the inorganic acid salt, the pH of the pharmaceutical composition is preferably in the range of 5.0 to 9.0. If the pH of the pharmaceutical composition of the present invention which contains the organic acid salt and/or the inorganic acid salt is less than 5.0 or more than 9.0, the physicochemical stability of the allergen is remarkably reduced, resulting in a safety problem. The pH is more preferably 6.0 to 8.0. The pharmaceutical composition of the present invention which contains the organic acid salt and/or the inorganic acid salt preferably contains the pH adjuster to control the pH in the above range.

The term "pH" herein refers to a value determined as follows.
In the case of measuring a liquid preparation, the pH of the liquid preparation is directly measured by a pH meter (e.g. a pH meter from HORIBA Ltd.) at 25°C ± 2°C.
In the case of measuring a solid preparation (including the case of a jelly preparation), a 1-g portion of the solid preparation is sampled in a 10-mL graduated flask, and diluted with distilled water. The contents are agitated at a constant temperature of 30°C to 35°C until the solid preparation is completely dissolved to give a sample solution. Then, the pH of the obtained sample solution is measured by a pH meter (e.g. a pH meter from HORIBA Ltd.) at 25°C ± 2°C.

Preferably, the pharmaceutical composition of the present invention further contains water.
In the case that the pharmaceutical composition of the present invention further contains water, the pharmaceutical composition can be prepared as a preferable jelly preparation described later. The reason for this is also described later.

Preferably, the pharmaceutical composition of the present invention contains gelatin.
The gelatin functions as a gelling agent or stabilizer, and examples thereof include those obtained by decomposing and extracting proteins in the skin and bones of animals with enzymes. For example, any of acid- or alkali-treated gelatins of porcine, bovine, or fish origin can be used.
The gelatin is preferably an alkali-treated gelatin in terms of the storage stability of the allergen, and is preferably a water-soluble gelatin in terms of the solubility thereof.
The gelatin is preferably gelatin of fish or porcine origin based on a consideration of the recent BSE problem.

In the case that the gelatin is added as a stabilizer, the amount thereof is preferably as much as possible but may be determined depending on the desired final dosage form.
For example, in the case that the pharmaceutical composition of the present invention is used as an injection, the amount of the gelatin is preferably 1 to 3% by weight. If the amount is less than 1% by weight, the allergen stabilization effect may be weak; if the amount is more than 3% by weight, the pharmaceutical composition may have a practical problem due to its viscosity.
In the case that the pharmaceutical composition of the present invention is used as a liquid preparation for oral administration, the amount of the gelatin is preferably 1 to 10% by weight. If the amount is less than 1% by weight, the allergen stabilization effect may be weak; if the amount is more than 10% by weight, the pharmaceutical composition may have a practical problem due to its viscosity.
In the case that the pharmaceutical composition of the present invention is used as a solid preparation for oral administration, the amount of the gelatin is preferably 1 to 80% by weight. If the amount is less than 1% by weight, the allergen stabilization effect may be weak; if the amount is more than 80% by weight, the pharmaceutical composition may not sufficiently release the allergen when orally administered, and therefore may not function enough.

Thus, the pharmaceutical composition of the present invention can be prepared as an injection, a liquid preparation for an oral administration, or a solid preparation for oral administration. In the case of any of these preparations, in addition to the above-mentioned ingredients, any of the following additives may be optionally used: excipients, binders, perfumes, flavoring substances, sweetening agents, colorants, antiseptics, antioxidants, stabilizers other than the organic acid salts and the inorganic acid salts, surfactants, and the like. These additives are not particularly limited and may be conventionally known ones.
Preferably, the dosage form of the pharmaceutical composition of the present invention is, for example, a solid preparation, a liquid preparation, or a jelly preparation.
Preferably, the pharmaceutical composition of the present invention is a liquid preparation for subcutaneous injection or oral administration.

Examples of the solid preparation for oral administration include tablets, coated tablets, powders, granules, microfine powders, oral rapid disintegration tablets, oral patches, jellies, and films, and it is not particularly limited, provided that it is a solid for oral administration, sublingual administration and buccal administration.

Preferably, the pharmaceutical composition of the present invention is for hyposensitization therapy.
The pharmaceutical composition of the present invention for hyposensitization therapy is preferably a jelly preparation.
Examples of the jelly preparation include those prepared by combining the pharmaceutical composition of the present invention described above, water, and gelatin as a gelling agent.
Such a jelly preparation is suited for oral hyposensitization therapy which requires control of the sensitization time, in particular, sublingual hyposensitization therapy. The jelly preparation can stably maintain allergens, in particular, proteins and peptides since it contains gelatin and a specific stabilizer.

The thickness of the jelly preparation is not particularly limited, and is preferably 30 to 5,000 µm. If the thickness is less than 30 µm, the jelly preparation may have problems of the sheet strength and handleability of a product; if the thickness is more than 5,000 µm, the jelly preparation may give unpleasant sensation when administered in the mouth, in particular, under the tongue.

The size of the jelly preparation is not particularly limited, but the jelly preparation preferably has a plane area within the range of 0.5 to 6.0 cm². If the plane area is less than 0.5 cm², the jelly preparation may be difficult to handle when picked up for administration; if the plane area is more than 6.0 cm², it may not be completely fit into the mouth, in particular, under the tongue.

The planar shape of the jelly preparation is not particularly limited, and may be any shape such as a polygonal shape (quadrangle (e.g. rectangle, square), pentagon, etc.), circle, and ellipse. The "polygonal shape" is intended to include polygonal shapes with slightly round corners as well as typical polygonal shapes.

The jelly preparation contains gelatin as a gelling agent.
The gelatin is a matrix forming material in the jelly preparation, and is edible and capable of maintaining the preparation in the film shape.

Since the jelly preparation contains gelatin, it is in the gel form at ambient temperature, and easily dissolves at approximately a body temperature inside the mouth. Gelatin turns into a gel at the lowest temperature among heat-reversible gelling agents, and enables drug production at ambient temperature to approximately 40°C. Namely, gelatin ensures the stability of heat-labile drugs during the production thereof.
The term "edible" herein means that the gelatin is orally administrable and is pharmaceutically acceptable.

The gelatin is preferably in a grade referred to as water-soluble gelatin which is soluble in water at ambient temperature. The use of such a water-soluble gelatin enables the jelly preparation to be produced at near ambient temperature and secures the safety during the production of drugs described below.
The term "water-soluble gelatin" herein refers to a gelatin that dissolves up to 1 g in 20 mL of water at ambient temperature (30°C).

Further, the gelatin, when formulated into a 10% by weight aqueous solution, preferably does not turn into a gel at 32°C but turns into a gel at near 5°C. This is because some gelatins having such a characteristic may sufficiently provide the effects of the present invention depending on the molecular weight and hydroxyproline content thereof even if they are not in the grade of water-soluble gelatins.

Examples of the gelatin used in the jelly preparation include those obtained by extracting and decomposing proteins in the skin and bones of animals with enzymes. For example, any of acid- or alkali-treated gelatins of porcine, bovine, or fish origin can be used.
The gelatin is particularly preferably gelatin of fish or porcine origin because it enables the preparation to be produced at ambient temperature and in terms of safety of an active ingredient during the production of the jelly preparation.
From such viewpoints, any gelatin may be adequate as long as it has an amino acid composition containing 5.2 to 9.2 mol% hydroxyproline and has an average molecular weight of exceeding 90,000. Examples of such a gelatin include those of fish origin such as salmon-derived gelatin (hydroxyproline content of amino acid sequence: 5.4 mol%), carp-derived gelatin (hydroxyproline content of amino acid sequence: 7.6 mol%), and tilapia-derived gelatin (hydroxyproline content of amino acid sequence: 8.0 mol%). Particularly preferable is tilapia-derived gelatin.

Here, the above-mentioned amino acid composition is analyzed using ninhydrin after hydrolyzation of gelatin and ion-exchange chromatographic separation.
Specific examples of the hydroxyproline content (mol%) of the amino acid sequence determined by the above-described method are as follows.
Fowl: 10.8 mol%
Ostrich: 10.4 mol%
Mouse: 8.7 mol%
Porcine:-9.4 mol%
Bovine: 9.5 mol%

In addition, any gelatin may be preferable regardless of the hydroxyproline content of the amino acid sequence, as long as it has an average molecular weight of 50,000 to 90,000.
The term "average molecular weight" herein means a weight average molecular weight measured by gel-filtration chromatography analysis.
Further, the average molecular weight herein means the average molecular weight of polypeptide chain monomers, not the average molecular weight of polypeptide chain trimers of gelatin.

In the jelly preparation, the amount of the gelatin is preferably 2 to 40% by weight, and more preferably 3 to 30% by weight, based on the total weight of the jelly preparation. If the amount is less than 2% by weight, the jelly preparation may not be in the gel form at ambient temperature; if the amount is more than 40% by weight, the jelly preparation may take a long time to dissolve in the mouth, which may cause a practical problem.

In addition to the edible polymer gelatin, the jelly preparation may further contain suitable amounts of edible polymers that are soluble only in water and/or edible polymers that dissolve neither in water nor in any organic solvents (hereafter, these are collectively referred to as other edible polymers), to the extent that they do not inhibit the effects of the present invention.
The amount of the other edible polymers is preferably 0.1 to 10% by weight based on the total weight of the jelly preparation.

The jelly preparation contains water.
The water aids in dissolution of the jelly preparation.
The dissolution time of the jelly preparation can be easily controlled by adjusting the water content of the jelly preparation. Therefore, the jelly preparation is suited for both administration manners that the preparation is dissolved in the mouth, and that the preparation is allowed to gradually dissolve in the mouth, in particular, under the tongue to gradually release the active ingredient.
In the present invention, the water content is preferably 1 to 60% by weight, and more preferably 5 to 50% by weight based on the total weight of the jelly preparation. If the water content is less than 1% by weight, the jelly preparation may hardly dissolve in the mouth, which may cause a practical problem; if the water content is more than 60% by weight, the physical properties of the jelly preparation may not be maintained stably during storage at ambient temperature.

The jelly preparation preferably further contains at least one additive selected from those that improve the physical properties and solubility of the jelly preparation (e.g. sugars, sugar alcohols, and sugar fatty acids).
Examples of such sugars include monosaccharides, disaccharides, and tri- to hexasaccharides listed below.
Examples of the monosaccharides include aldotetroses such as erythrose and threose; aldopentoses such as ribose, lyxose, xylose, and arabinose; aldohexoses such as allose, talose, gulose, glucose, altrose, mannose, galactose, and idose; ketotetroses such as erythrulose; ketopentoses such as xylulose and ribulose; and ketohexoses such as psicose, fructose, sorbose, and tagatose. Examples of the disaccharides include α-diglucosides such as trehalose, kojibiose, nigerose, maltose, and isomaltose; β-diglucosides such as isotrehalose, sophorose, laminaribiose, cellobiose, and genthiobiose; α,β-diglucosides such as neotrehalose; and lactose, sucrose, and isomaltulose (palatinose). Examples of the trisaccharides include raffinose. Examples of the tri- to hexasaccharides include oligosaccharides such as cyclic oligosaccharides including fructooligosaccharide, galactooligosaccharide, xylooligosaccharide, isomaltooligosaccharide, chitin oligosaccharides, chitosan oligosaccharides, oligoglucosamine, dextrin, and cyclodextrin.

Examples of sugar alcohols of monosaccharide include tetritols such as erythritol, D-threitol, and L-threitol; pentitols such as D-arabinitol and xylitol; hexitols such as D-iditol, galactitol (dulcitol), D-glucitol (sorbitol), and mannitol; and cyclitols such as inositol. Examples of sugar alcohols of disaccharides include maltitol, lactitol, and reduced palatinose (isomalt). Examples of sugar alcohols of oligosaccharides include pentaerythritol and reduced maltose syrup.
The jelly preparation may contain one or two or more of these sugars and sugar alcohols, and these sugars and sugar alcohols may be substituted.

In order to aid in dissolution of the jelly preparation in the mouth and to avoid a great change in the viscosity of the solution during the production, mono- to trisaccharides or sugar alcohols thereof are preferably used.

Examples of the sugar fatty acids include sorbitan fatty acid esters and sucrose fatty acid esters.
Examples of the sorbitan fatty acid esters include sorbitan monooleate, sorbitan trioleate, sorbitan sesquioleate, sorbitan cocoate, and polyoxyethylene sorbitan fatty acid esters.
Examples of the sucrose fatty acid esters include sucrose stearate, sucrose oleate, sucrose palmitate, sucrose myristate, sucrose behenate, sucrose erucate, and sucrose-mixed fatty acid esters.
Advantageously, these sugar fatty acids function as antifoamers as well as function as stabilizers for proteins and peptides.

The jelly preparation preferably contains polyethylene glycol, a derivative thereof, or cellulose, which are additives that improve the physical properties of the jelly preparation.
The polyethylene glycol preferably has an average molecular weight of 200 to 20,000, and more preferably 400 to 8,000. If the average molecular weight is less than 200, the polyethylene glycol has too high plasticity and may not provide sufficient levels of physical properties required for practical use; if the average molecular weight is more than 20,000, the jelly preparation may become too viscous and give an unpleasant sensation when dissolved in the mouth. The average molecular weight herein is determined by the average molecular weight test specified in the Japanese Pharmacopoeia, the 15th edition, the section of macrogol 400.

The cellulose is preferably crystalline cellulose or powdery cellulose, and is more preferably crystalline cellulose.
The cellulose preferably has an average particle size of 0.01 to 100 µm, and more preferably 0.01 to 50 µm. If the average particle size is less than 0.01 µm, the cellulose tends to aggregate in the solution during the production, thereby resulting in poor physical properties. If the average particle size is more than 100 µm, the cellulose tends to be precipitated in the solution during the production, and a portion thereof may remain and give an unpleasant sensation when the jelly preparation is administered in the mouth. The average particle size herein means a 50% average particle size determined by a laser scattering particle-size distribution analyzer.

The amount of the additive in the jelly preparation is preferably 1 to 80% by weight, and more preferably 5 to 70% by weight based on the total weight of the jelly preparation. If the amount is less than 1% by weight, sufficient levels of the physical properties required for practical use may not be secured; if the amount is more than 80% by weight, the additive added may make it difficult to control the physical properties of the jelly preparation.

The jelly preparation may optionally contain, as matrix forming materials, any of perfumes, flavoring substances, sweetening agents, colorants, antiseptics, antioxidants, other stabilizers, surfactants, and the like, in addition to the ingredients mentioned above. These ingredients are not particularly limited and may be conventionally known ones.

As described above, due to the presence of the gelatin, the jelly preparation is in the gel form at ambient temperature and easily dissolves at approximately a body temperature inside the mouth. The use of the specific additive in combination with the gelatin significantly improves the physical properties advantageous for practical use. The jelly preparation can stably maintain an allergenic protein, in particular, the cedar pollen allergen protein.
Since the dissolution time of the jelly preparation can be easily controlled by adjusting the water content, the jelly preparation is suitable for oral hyposensitization therapy which requires control of the sensitization time, in particular, sublingual hyposensitization therapy.
Of course, the jelly preparation may be swallowed as it is, or may be immediately dissolved in the mouth and then swallowed. Alternatively, the dissolution time in the mouth may be controlled so that the active ingredient is absorbed through oral mucosa including sublingual mucosa. The jelly preparation remarkably improves the QOL of patients and caregivers because of the following advantages: the jelly preparation completely dissolves at approximately a body temperature, and therefore does not give unpleasant sensation due to residues; and since the jelly preparation is in the sheet form and has a larger surface area than those of other dosage forms such as tablets, patients and caregivers can easily pick it up with fingers.

The jelly preparation can be produced by, for example, a method including preparing a mixed solution by mixing water, the gelatin, the organic acid salt and/or the inorganic acid salt, and the allergen, and forming a thin film of the mixed solution. In this method, the water content of the resulting pharmaceutical composition is controlled by adjusting the amount of water to be added in the step of preparing a mixed solution, or by drying the thin film after the step of forming a thin film.

In the step of preparing a mixed solution, for example, the gelatin, the organic acid salt and/or the inorganic acid salt, and optionally other additives are dissolved in a predetermined amount of water at ambient temperature or under heat, and insoluble additives are homogeneously dispersed therein. If the allergen is heat-stable, the mixed solution may be prepared by adding the allergen simultaneously with other ingredients including the gelatin. If the allergen is heat-labile, the mixed solution may be prepared by preparing a gelatin solution by dissolving the ingredients including gelatin; cooling the gelatin solution to a temperature near ambient temperature to 35°C; and adding the allergen thereto and agitating the resulting mixture. Alternatively, the allergen may be added in a later-described step of dispensing the mixed solution or spreading the mixed solution.
Even if bubbles occur during the preparation of the mixed solution, they will disappear, for example, after leaving the solution overnight or by vacuum or reduced pressure degassing.

In the step of forming a thin film, for instance, a predetermined amount of the mixed solution is dispensed into a plastic blister case of a desired size at a temperature of 28°C to 32°C, and cool-solidified into a thin film immediately after the dispensation. In lieu of the dispensation method, a suitable amount of the mixed solution may be spread over a release film and cool-solidified to form a thin film, and then the film may be cut into a desired size.
The thin film formed in this step preferably has the same size as the jelly preparation.

In the method for producing the jelly preparation, the water content of the jelly preparation is controlled by adjusting the amount of water to be added in the step of preparing a mixed solution or by drying the thin film after the step of forming a thin film.
That is, in the case of adjusting the water content in the step of preparing a mixed solution by adjusting the amount of water to be added, the jelly preparation can be obtained as the thin film.
On the other hand, in the case of adjusting the water content after the step of forming a thin film by drying the thin film, the jelly preparation can be completed by drying the thin film, as described above.
Examples of the method for drying the thin film include a method including a cold air drying step or a reduced pressure cool drying step.

Since at least one of the organic acid salt, the inorganic acid salt, and the pH adjuster which improve the heat stability of the allergen is used, the method for producing the jelly preparation has the following remarkable advantages: a loss due to inactivation of the allergen during the production can be avoided; and an allergenic protein, in particular, a heat-labile allergenic protein can be formulated into a drug at high temperatures (ambient temperature to 40°C).
Preferably, the resulting pharmaceutical composition is optionally sealed in a package and is thus prepared as a product.

Alternatively, the pharmaceutical composition of the present invention preferably does not contain water.
In the case that the pharmaceutical composition of the present invention does not contain water, for example, it has an advantage in terms of production costs because the pharmaceutical composition does not require sterilization treatment and antiseptics which are necessary for conventional jelly preparations of pharmaceutical compositions. In addition, it is suitably used in nutritious supplements for patients who require a fluid restriction.
The wording "does not contain water" herein is intended to mean that substantially no water is contained, and specifically means that the water content is not more than 5% by weight, preferably not more than 2.5% by weight, and more preferably not more than 1% by weight based on the total weight of the pharmaceutical composition of the present invention.

In the case that the pharmaceutical composition of the present invention does not contain water, the pharmaceutical composition of the present invention can be in the form of a porous solid containing the gelatin, which is called as cake. The pharmaceutical composition of the present invention is preferably a lyophilized preparation produced by sublimating the solvent water from an allergen-containing aqueous solution by lyophilization.
Such a lyophilized preparation of the pharmaceutical composition of the present invention is physically stable at a temperature of ambient temperature to about 60°C.
Since the matrix of the pharmaceutical composition of the present invention is mainly composed of the gelatin, the pharmaceutical composition is easily dissolved at approximately a body temperature inside the mouth with water in the mouth. If the pharmaceutical composition contains the specific additive as well, the physical properties advantageous for practical use are significantly improved.
In addition, the pharmaceutical composition can stably maintain an allergenic protein, in particular, the cedar pollen allergen protein.
Of course, the pharmaceutical composition of the present invention may be swallowed as it is, or may be immediately dissolved in the mouth and then swallowed. Alternatively, the dissolution time in the mouth may be controlled so that the active ingredient is absorbed through oral mucosa including sublingual mucosa.
The pharmaceutical composition of the present invention remarkably improves the QOL of patients and caregivers because of the following advantages: it completely dissolves at approximately a body temperature without residues left; it is physically stable; and patients and caregivers can easily pick it up with fingers.

The physical strength of the pharmaceutical composition of the present invention is not particularly limited, but is preferably enough to avoid physical damage such as cracks and fractions in a drug product thereof when packed, stored, transported, or handled by a patient. The pharmaceutical composition does not melt or show a deterioration of the properties at all when touched with hands, that is, touched at approximately a body temperature.
Drug products produced using the pharmaceutical composition of the present invention are physically stable, but are required to have a property to break down immediately in the presence of water, for example, in contact with saliva in the mouth. The breakdown time in the mouth is preferably not longer than 90 seconds, and more preferably not longer than 60 seconds.

The size of the pharmaceutical composition of the present invention is not particularly limited, but the pharmaceutical composition preferably has a plane area of 0.5 to 6.0 cm². If the area is less than 0.5 cm², a drug product of the pharmaceutical composition of the present invention may be difficult to handle when picked up with fingers for administration; if the area is more than 6.0 cm², it may not be completely fit into the mouth, in particular, under the tongue.

The pharmaceutical composition of the present invention can be prepared, for example, by a method that includes dissolving, in water, the allergen and at least one selected from the group consisting of the organic acid salt, the inorganic acid salt, and the pH adjuster, thereby providing an allergen-containing aqueous solution, and lyophilizing the allergen-containing aqueous solution.
Such a method for preparing the pharmaceutical composition of the present invention is also one aspect of the present invention.

The method for preparing the pharmaceutical composition of the present invention includes preparing an allergen-containing aqueous solution.
In this step, for example, at least one selected from the group consisting of the organic acid salt, the inorganic acid salt, and the pH adjuster, and optionally other additives are dissolved in a predetermined amount of water at ambient temperature or under heat, and insoluble additives are homogeneously dispersed therein. If the allergen is heat-stable, the allergen-containing aqueous solution is prepared by adding the allergen simultaneously with other ingredients such as the organic acid salt. If the allergen is heat-labile, the allergen-containing aqueous solution is prepared by preparing a solution by dissolving the ingredients such as the organic acid salt; cooling the solution to a temperature near ambient temperature to 35°C; and adding the allergen thereto and agitating the resulting mixture. Alternatively, the allergen may be added in a later-described step of dispensing the allergen-containing aqueous solution or spreading the allergen-containing aqueous solution.
Even if bubbles occur during the preparation of the allergen-containing aqueous solution, they will disappear, for example, after leaving the solution overnight or by vacuum or reduced pressure degassing.

In the step of preparing an allergen-containing aqueous solution, the gelatin is also preferably dissolved in the allergen-containing aqueous solution. In the case that the gelatin is dissolved in the allergen-containing aqueous solution, the gelatin preparation can be obtained.

The allergen-containing aqueous solution prepared in the step of preparing an allergen-containing aqueous solution is preferably adjusted to a pH of 5.5 to 8.5. If the allergen-containing aqueous solution contains the organic acid salt and/or the inorganic acid salt, the pH is preferably 5.0 to 9.0.
If the allergen-containing aqueous solution has a pH in the above range, it is possible to avoid a great deterioration of the physicochemical stability of the allergen and to secure the safety.
Examples of a method for adjusting the pH of the allergen-containing aqueous solution in the above range include a method of adjusting the amount of the pH adjuster to be added.

Preferably, in the step of lyophilizing the allergen-containing aqueous solution, for instance, a predetermined amount of the allergen-containing aqueous solution is dispensed into a blister of a desired size for lyophilization at a temperature of 28°C to 35°C, and lyophilized immediately after the dispensation.
Preferably, the resulting pharmaceutical composition is optionally sealed in a package and is thus prepared as a product.

The pharmaceutical composition of the present invention prepared by the above method is, as described above, a lyophilized preparation, and therefore is a solid preparation suited for oral administration, but can be also formulated as an injection or preparation for transmucosal (transnasal, oral, sublingual) administration because the pharmaceutical composition is excellent in usability after lyophilization and solubility in water for injection and can maintain the stability of the allergen for a long period of time.

The pharmaceutical composition of the present invention contains an allergen and at least one stabilizer selected from the group consisting of an organic acid salt, an inorganic acid salt, and a pH adjuster. Therefore, the pharmaceutical composition of the present invention allows stable preservation and delivery of allergens, which are known to be heat-labile. The pharmaceutical composition of the present invention can be used for an injection or a liquid preparation for oral administration, and also can be formulated as a stable solid preparation for oral administration in addition to these liquid forms if gelatin is used as a gelling agent.
If the pharmaceutical composition of the present invention contains gelatin, the pharmaceutical composition can be formulated was a jelly preparation. Such a jelly preparation remarkably improves the QOL of patients and caregivers because of the following advantages: it can be administered by a patient himself/herself without pain unlike injections; it can be divided and therefore allows control of the dose; it is very portable; it does not give unpleasant sensation due to residues and prevents accidental intake because of its different shape from that of tablets; and a caregiver can easily administer it to a patient.
Since the jelly preparation contains the organic acid salt and/or the inorganic acid salt as stabilizer(s), or contains a pH adjuster to adjust the pH, allergens, which are known to be heat-labile, can be stably maintained during the production thereof.

The following description is given to illustrate the present invention by way of examples, but the present invention is not limited to these examples.

### (Experimental Examples 1 to 4)

Water-soluble gelatin (fish origin) (10 parts by weight, CSF from Nippi Inc.) was added to pure water (860 parts by weight) and dissolved therein at a temperature of 30°C to 40°C. After the dissolution, the solution was recovered to room temperature. At this point, no gelation was observed in the solution. Separately, cedar pollen extract dry powder (0.1 parts by weight, from LSL Co., Ltd.) was added to pure water (20 parts by weight), and dissolved therein at room temperature. This solution was combined with the whole gelatin solution prepared above, and the resulting mixture was immediately agitated. No gelation was observed in the mixture.
The mixture was adjusted to a pH shown in Table 1 with a pH adjuster (sodium hydroxide), and combined with pure water so that an allergen-containing gelatin aqueous solution (1,000 parts by weight in total) was obtained. The resulting allergen-containing gelatin aqueous solution was agitated on a shaker at 35°C, and measured for allergen activity after 30 minutes and 60 minutes by the method described below.
Subsequently, the allergen-containing gelatin aqueous solution was recovered to room temperature. At this point, no gelation was observed in the solution. Then, a 1.0-g portion thereof was immediately poured into a vial for lyophilization and lyophilized. In this manner, medicament-containing compositions were prepared respectively. These medicament-containing compositions were evaluated by a storage stability test in which the medicament-containing compositions were stored for 14 days at 40°C ± 2°C, and measured for allergen activity after 7 days and 14 days by the method described below. Table 2 shows the results.

### (Experimental Examples 5 to 12)

Lyophilized medicament-containing compositions were prepared respectively in the same manner as in Experimental Examples 1 to 4, except that the following gelatins were used: porcine bone gelatin (AEP from Nippi Inc.) in Experimental Examples 5 to 8; and alkali-treated bovine gelatin (AD4 from Nippi Inc.) in Experimental Examples 9 to 12. The pH was adjusted to the values shown in Table 1 with an appropriate pH adjuster. The allergen-containing gelatin aqueous solutions and the compositions after the storage stability test were measured for allergen activity in the same manner as in Experimental Examples 1 to 4.

### (Comparative Experimental Examples 1 to 6)

Lyophilized medicament-containing compositions were prepared respectively in the same manner as in Experimental Examples 1 to 4, except that the following gelatins were used: water-soluble gelatin (fish origin) (CSF from Nippi Inc.) in Comparative Experimental Examples 1 and 2; porcine bone gelatin (AEP from Nippi Inc.) in Comparative Experimental Examples 3 and 4; and alkali-treated bovine gelatin (AD4 from Nippi Inc.) in Comparative Experimental Examples 5 and 6. The pH was adjusted to the values shown in Table 1 with an appropriate pH adjuster. The allergen-containing gelatin aqueous solutions and the compositions after the storage stability test were measured for allergen activity in the same manner as in Experimental Examples 1 to 4.

**[Table 1]**

| Ingredient | Amount [parts by weight] | | | | | | | | | | | | | | | | | |
|---|---|---|---|---|---|---|---|---|---|---|---|---|---|---|---|---|---|---|
| | Experimental Example | | | | | | | | | | | | Comparative Experimental Example | | | | | |
| | 1 | 2 | 3 | 4 | 5 | 6 | 7 | 8 | 9 | 10 | 11 | 12 | 1 | 2 | 3 | 4 | 5 | 6 |
| Cedar pollen extract dry powder | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Water-soluble gelatin (fish origin) | 10 | 10 | 10 | 10 | - | - | - | - | - | - | - | - | 10 | 10 | - | - | - | - |
| Porcine bone gelatin | - | - | - | - | 10 | 10 | 10 | 10 | - | - | - | - | - | - | 10 | 10 | - | - |
| Alkali-treated bovine gelatin | - | - | - | - | - | - | - | - | 10 | 10 | 10 | 10 | - | - | - | - | 10 | 10 |
| pH | 5 | 6 | 8 | 9 | 5 | 6 | 8 | 9 | 5 | 6 | 8 | 9 | 4 | 10 | 4 | 10 | 4 | 10 |

### (Allergen activity evaluation method)

The allergen activity of Cry j 1, one of the major allergens of cedar pollens, was measured using a cedar pollen antigen ELISA Kit "Cry j1" (from Seikagaku Biobusiness Corp.). The principle of the measurement kit is a sandwich ELISA method that utilizes monoclonal antibodies (013, 053) specific to Cry j 1, which is one of the Japanese Cedar (Cryptomeria japonica) pollen antigens, and the method allows specific Cry j 1 measurement.
To 100 µL of a reaction buffer solution included in the kit was added 20 µL of a standard solution or a sample, and a primary reaction was carried out at ambient temperature for 60 minutes. Then, 100 µL of an HRP-labeled antibody solution was added thereto and a secondary reaction was carried out for 60 minutes. Added thereto was 100 µL of an enzyme substrate solution, and a reaction was carried out for 30 minutes at ambient temperature while light was shielded. Finally, 100 µL of a reaction stop solution was added thereto. Thereafter, the ultraviolet absorption intensity at 450 nm was measured. A calibration curve was determined based on the absorption intensity of the standard solution at various Cry j 1 concentrations, and the Cry j 1 allergen activity (ng/mL) of each sample was determined based on the calibration curve.
The Cry j 1 allergen activity % was determined for the samples under and after the storage stability test (7 days and 14 days after) and the allergen-containing gelatin aqueous solutions immediately after the production (30 minutes and 60 minutes after). The Cry j 1 allergen activity was evaluated based on the following scoring criteria.
5: not less than 90% and less than 105%
4: not less than 75% and less than 90%
3: not less than 60% and less than 75%
2: not less than 45% and less than 60%
1: not less than 30% and less than 45%
0: less than 30%

**[Table 2]**

| Sample | Remaining allergen activity | | | |
|---|---|---|---|---|
| | Solution [35°C] | | Lyophilized com | position [40°C] |
| | 30 minutes after | 60 minutes after | 7 days after | 14 days after |
| Experimental Example 1 | 5 | 4 | 4 | 4 |
| Experimental Example 2 | 5 | 5 | 5 | 5 |
| Experimental Example 3 | 5 | 5 | 5 | 5 |
| Experimental Example 4 | 5 | 4 | 4 | 4 |
| Experimental Example 5 | 5 | 4 | 4 | 4 |
| Experimental Example 6 | 5 | 5 | 5 | 5 |
| Experimental Example | 5 | 5 | 5 | 5 |
| Experimental Example 8 | 5 | 4 | 4 | 4 |
| Experimental Example 9 | 5 | 4 | 4 | 4 |
| Experimental Example 10 | 5 | 5 | 5 | 5 |
| Experimental Example 11 | 5 | 5 | 5 | 5 |
| Experimental Example 12 | 5 | 4 | 4 | 4 |
| Comparative Experimental Example 1 | 3 | 2 | 2 | 2 |
| Comparative Experimental Example 2 | 3 | 2 | 2 | 2 |
| Comparative Experimental Example 3 | 3 | 2 | 2 | 2 |
| Comparative Experimental Example 4 | 3 | 2 | 2 | 2 |
| Comparative Experimental Example 5 | 3 | 2 | 2 | 2 |
| Comparative Experimental Example 6 | 3 | 2 | 2 | 2 |

Table 2 shows that the optimal pH range for the cedar pollen allergen is 5.0 to 9.0, and more preferably 6.0 to 8.0.
The results also suggest that if the pH is not more than 4.0 or not less than 10.0, the cedar pollen allergen is deactivated soon. All the lyophilized products of the sample solutions did not show a great reduction in the allergen potency after the 14-day storage at 40°C.

### (Experimental Example 13)

Water-soluble gelatin (fish origin) (10 parts by weight, CSF from Nippi Inc.) was added to pure water (860 parts by weight) and dissolved therein at a temperature of 30°C to 40°C. After the dissolution, the solution was recovered to room temperature. At this point, no gelation was observed in the solution. Separately, cedar pollen extract dry powder (0.1 parts by weight, from LSL Co., Ltd.) was added to pure water (20 parts by weight) and dissolved therein at room temperature. This solution was combined with the whole gelatin solution prepared above, and the resulting mixture was immediately agitated. No gelation was observed in the mixture. The mixture was adjusted to pH 7 with a pH adjuster (sodium hydroxide), and combined with pure water so that an allergen-containing gelatin aqueous solution (1,000 parts by weight in total) was obtained. Then, a 1.0-g portion thereof was immediately poured into a vial for lyophilization and lyophilized. In this manner, a medicament-containing composition was obtained. The medicament-containing composition was stored at 40°C ± 2°C, and measured for allergen activity after 7 days, 14 days, 30 days, and 120 days by the method described above. Table 4 shows the results.

### (Experimental Examples 14 to 20)

Medicament-containing compositions were prepared in the same manner as in Experimental Example 13, except that the following gelatins were used: fish gelatin (FGS-230 from Nippi Inc.) in Experimental Example 14; water-soluble gelatin (porcine origin) (CS from Nippi Inc.) in Experimental Example 15; acid-treated porcine gelatin (AP-200F from Nippi Inc.) in Experimental Example 16; alkali-treated porcine gelatin (BP-200F from Nippi Inc.) in Experimental Example 17; porcine bone gelatin (AEP from Nippi Inc.) in Experimental Example 18; bovine gelatin (CP-1045 from JELLICE) in Experimental Example 19; and alkali-treated bovine gelatin (AD4 from Nippi Inc.) in Experimental Example 20.

**[Table 3]**

| Ingredient | Amount [parts by weight] | | | | | | | |
|---|---|---|---|---|---|---|---|---|
| | Experimental Example | | | | | | | |
| | 13 | 14 | 15 | 16 | 17 | 18 | 19 | 20 |
| Cedar pollen extract dry powder | 0.1 | 0.1 | 0.1 | 0.1 | 0.1 | 0.1 | 0.1 | 0.1 |
| Water-soluble gelatin (fish origin) | 10 | - | - | - | - | - | - | - |
| Fish gelatin | - | 10 | - | - | - | - | - | - |
| Water-soluble gelatin (porcine origin) | - | - | 10 | - | - | - | - | - |
| Acid-treated porcine gelatin | - | - | - | 10 | - | - | - | - |
| Alkafi-treated porcine gelatin, | - | - | - | - | 10 | - | - | - |
| Porcine bone gelatin | - | | - | - | - | 10 | - | - |
| Bovine gelatin | - | - | - | - | - | - | 10 | - |
| Alkali-treated bovine gelatin | - | - | - | - | - | - | - | 10 |
| PEG 4000 | - | - | - | - | - | - | - | - |
| PEG 6000 | - | - | - | - | - | - | - | - |
| EG 20000 | - | - | - | - | - | - | - | - |
| Mannitol | - | - | - | - | - | - | - | - |

**[Table 4]**

| Sample | Remaining allergen activity | | | |
|---|---|---|---|---|
| | 7 days after | 14 days after | 30 days after | 120 days after |
| Experimental Example 13 | 5 | 5 | 4 | 3 |
| Experimental Example 14 | 5 | 5 | 4 | 3 |
| Experimental Example 15 | 5 | 5 | 4 | 3 |
| Experimental Example 16 | 5 | 5 | 4 | 3 |
| Experimental Example 17 | 5 | 5 | 4 | 3 |
| Experimental Example 18 | 5 | 5 | 4 | 3 |
| Experimental Example 19 | 5 | 5 | 4 | 3 |
| Experimental Example 20 | 5 | 5 | 4 | 3 |

Table 4 shows that the medicament-containing compositions of Experimental Examples which contain gelatin as a matrix did not show a great reduction in the allergen activity. The results also demonstrate that any of the gelatins stabilizes the cedar pollen allergen. However, due to lack of the organic acid salt, they showed a slight reduction in the allergen activity after a long period of time (120 days).

### (Experimental Example 21)

Calcium lactate (10 parts by weight, calcium lactate hydrate (granules) from Taihei Chemical Industrial Co., Ltd.) was added to pure water (960 parts by weight) and dissolved therein at room temperature. Separately, cedar pollen extract dry powder (0.1 parts by weight, from LSL Co., Ltd.) was added to pure water (20 parts by weight) and dissolved therein at room temperature. This solution was combined with the whole former solution prepared above, and the resulting mixture was immediately agitated. In this manner, a medicament-containing aqueous solution was prepared. This medicament-containing aqueous solution was measured for pH. Then, a 1.0-g portion of the medicament-containing aqueous solution was poured into a vial for lyophilization and lyophilized. In this manner, a medicament-containing composition was obtained. The medicament-containing composition was stored at 40°C ± 2°C and measured for allergen activity after 7 days and 14 days. Table 6 shows the results.

### (Experimental Examples 22 to 31 and Comparative Experimental Examples 7 and 8)

As shown in Table 5, medicament-containing compositions were prepared in the same manner as in Experimental Example 21, except that the following additives were respectively used: dipotassium glycyrrhizate (from Wako Pure Chemical Industries, Ltd.), sodium citrate (from Wako Pure Chemical Industries, Ltd.), sodium malate (disodium DL-malate n-hydrate from Wako Pure Chemical Industries, Ltd.), calcium gluconate (from Wako Pure Chemical Industries, Ltd.), disodium succinate (from Wako Pure Chemical Industries, Ltd.), potassium sodium tartrate (potassium sodium tartrate tetrahydrate from Wako Pure Chemical Industries, Ltd.), sodium tartrate (sodium L-tartrate from Wako Pure Chemical Industries, Ltd.), sodium L-ascorbate (from Wako Pure Chemical Industries, Ltd.), sodium gluconate (from Fuso Chemical Co., Ltd.), sodium L-aspartate (from Kyowa Hakko Bio Co., Ltd.), potassium hydrogen tartrate (potassium hydrogen L-tartrate from Komatsuya), and monosodium fumarate (MONOFUMAR from Nippon Shokubai Co., Ltd.).

**[Table 5]**

| Ingredient | Amount [parts by weight] | | | | | | | | | | | | |
|---|---|---|---|---|---|---|---|---|---|---|---|---|---|
| | Experimental Example | | | | | | | | | | | Comparative Experimental Example | |
| | 21 | 22 | 23 | 24 | 25 | 26 | 27 | 28 | 29 | 30 | 31 | 7 | 8 |
| Cedar pollen extract dry powder | 0.1 | 0.1 | 0.1 | 0.1 | 0.1 | 0.1 | 0.1 | 0.1 | 0.1 | 0.1 | 0.1 | 0.1 | 0.1 |
| Calcium lactate | 10 | - | - | - | - | - | - | - | - | - | - | - | - |
| Dipotassium glycyrrhizate | - | 10 | - | - | - | - | - | - | - | - | - | - | - |
| Sodium citrate | - | - | 10 | - | - | - | - | - | - | - | - | - | - |
| Sodium malate | - | - | - | 10 | - | - | - | - | - | - | - | - | - |
| Calcium gluconate | - | - | - | - | 10 | - | - | - | - | - | - | - | - |
| Disodium succinate | - | - | - | - | - | 10 | - | - | - | - | - | - | - |
| Potassium sodium tartrate | - | - | - | - | - | - | 10 | - | - | - | - | - | - |
| Sodium tartrate | - | - | - | - | - | - | - | 10 | - | - | - | - | - |
| Sodium L-ascorbate | - | | - | - | - | - | - | - | 10 | - | - | - | - |
| Sodium gluconate | - | - | - | - | - | - | - | - | - | 10 | - | - | - |
| Sodium L-aspartate | - | - | - | - | - | - | - | - | - | - | 10 | - | - |
| Potassium hydrogen tartrate | - | - | - | - | - | - | - | - | - | - | - | 10 | - |
| Monosodium fumarate | - | - | - | - | - | - | - | - | - | - | - | - | 10 |

**[Table 6]**

| Organic acid salt | Sample | pH | Remaining allergen activity | |
|---|---|---|---|---|
| | | | 7 days after | 14 days after |
| Calcium lactate | Experimenta Example 21 | 6.8 | 5 | 5 |
| Dipotassium glycyrrhizate | Experimenta Example 22 | 5.1 | 5 | 5 |
| Sodium citrate | Experimenta Example 23 | 8.3 | 5 | 5 |
| Sodium malate | Experimenta Example 24 | 6.9 | 5 | 4 |
| Calcium gluconate | Experimenta Example 25 | 6.6 | 5 | 5 |
| Disodium succinate | Experimenta Example 26 | 7.9 | 4 | 4 |
| Potassium sodium tartrate | Experimenta Example 27 | 7.1 | 4 | 4 |
| Sodium L-tartrate | Experimenta Example 28 | 7.2 | 5 | 4 |
| Sodium L-ascorbate | Experimenta Example 29 | 7.3 | 4 | 4 |
| Sodium gluconate | Experimenta Example 30 | 6.8 | 5 | 5 |
| Sodium L-aspartate | Experimenta Example 31 | 6.7 | 5 | 5 |
| Potassium hydrogen tartrate | Comparative Experimental Example 7 | 3.5 | 2 | 1 |
| Monosodium fumarate | Comparative Experimental Example 8 | 3.6 | 2 | 1 |

Table 6 shows that the organic acid salts that are capable of adjusting the pH (of the 1% by weight medicament-containing aqueous solutions) in the range of 5.0 to 9.0 provide high stability of the allergen. In particular, calcium lactate, dipotassium glycyrrhizate, sodium citrate, calcium gluconate, sodium gluconate, and sodium L-aspartate provide higher allergen stability.
The results also demonstrate that although the most preferable pH range for the cedar pollen allergen is 6 to 8, not all of the organic acid salts that provide high stability adjust the pH in this optimal pH range, and that organic acid salts may provide different levels of stability even if they adjust the pH to the same level.
Accordingly, the results revealed that the allergen stabilization effect of the organic acid salts does not always depend on the pH of the aqueous solutions.

### (Example 1)

A gelatin aqueous solution was prepared by adding water-soluble gelatin (fish origin) (10 parts by weight, CSF from Nippi Inc.) and calcium lactate (5 parts by weight, calcium lactate hydrate (granules) from Taihei Chemical Industrial Co., Ltd.) to pure water (850 parts by weight), and dissolving them at a temperature of 30°C to 40°C. After the dissolution, the solution was recovered to room temperature. At this point, no gelation was observed in the solution. Separately, cedar pollen extract dry powder (0.1 parts by weight, from LSL Co., Ltd.) was added to pure water (20 parts by weight) and dissolved therein at room temperature. This solution was combined with the whole gelatin solution prepared as described above, and the resulting mixture was immediately agitated. At this point, no gelation was observed in the mixture. The solution was adjusted to pH 7 with a pH adjuster (sodium hydroxide), and combined with pure water so that an allergen-containing gelatin aqueous solution (1,000 parts by weight in total) was obtained. Then, 1.0-g portions thereof were poured into vials for lyophilization and lyophilized. In this manner, a pharmaceutical composition was obtained. The pharmaceutical composition was picked up with fingers to evaluate its suitability for practical use.
Next, 5.0 g of pure water at 5°C, 25°C, or 30°C was poured to the pharmaceutical composition, and the dissolving performance of the pharmaceutical composition was observed at room temperature, and evaluated based on the following criteria. Another portion of the pharmaceutical composition was stored at 40°C ± 2°C, and measured for allergen activity after 30 days, 60 days, 90 days, and 120 days. Tables 8 and 9 show the results.
++: shorter than 30 seconds until complete dissolution
+: about 30 seconds to 1 minute until complete dissolution
±: 1 minute or longer until complete dissolution

### (Examples 2 to 11)

As shown in Table 7, pharmaceutical compositions were prepared in the same manner as in Example 1, except that the following organic acid salts were respectively used: dipotassium glycyrrhizate (from Wako Pure Chemical Industries, Ltd.), sodium citrate (from Wako Pure Chemical Industries, Ltd.), sodium gluconate (from Fuso Chemical Co., Ltd.), sodium L-aspartate (from Kyowa Hakko Bio Co., Ltd.), sodium malate (disodium DL-malate n-hydrate from Wako Pure Chemical Industries, Ltd.), calcium gluconate (from Wako Pure Chemical Industries, Ltd.), disodium succinate (from Wako Pure Chemical Industries, Ltd.), potassium sodium tartrate (potassium sodium tartrate tetrahydrate from Wako Pure Chemical Industries, Ltd.), sodium tartrate (sodium L-tartrate from Wako Pure Chemical Industries, Ltd.), and sodium L-ascorbate (from Wako Pure Chemical Industries, Ltd.). The pharmaceutical compositions were evaluated in the same manner as in Example 1.

### (Examples 12 to 22)

As shown in Table 7, pharmaceutical compositions were prepared respectively in the same manner as in Example 1. However, the gelatin used was porcine bone gelatin (AEP from Nippi Inc.), and the organic acid salts used were calcium lactate (calcium lactate hydrate (granules) from Taihei Chemical Industrial Co., Ltd.), dipotassium glycyrrhizate (from Wako Pure Chemical Industries, Ltd.), sodium citrate (from Wako Pure Chemical Industries, Ltd.), sodium gluconate (from Fuso Chemical Co., Ltd.), sodium L-aspartate (from Kyowa Hakko Bio Co., Ltd.), sodium malate (disodium DL-malate n-hydrate from Wako Pure Chemical Industries, Ltd.), calcium gluconate (from Wako Pure Chemical Industries, Ltd.), disodium succinate (from Wako Pure Chemical Industries, Ltd.), potassium sodium tartrate (potassium sodium tartrate tetrahydrate from Wako Pure Chemical Industries, Ltd.), sodium tartrate (sodium L-tartrate from Wako Pure Chemical Industries, Ltd.), and sodium L-ascorbate (from Wako Pure Chemical Industries, Ltd.), respectively. The pharmaceutical compositions were evaluated in the same manner as in Example 1. The pH adjuster used to adjust the pH in Example 14 was hydrochloric acid.

**[Table 7]**

| Ingredient | Amount [parts by weight] | | | | | | | | | | | | | | | | | | | | | |
|---|---|---|---|---|---|---|---|---|---|---|---|---|---|---|---|---|---|---|---|---|---|---|
| | Example | | | | | | | | | | | | | | | | | | | | | |
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 |
| Cedar pollen extract dry powder | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Watar-soluble gelatin (fish origin) | 10 | 10 | 10 | 10 | 10 | 10 | 10 | 10 | 10 | 10 | 10 | - | - | - | - | - | - | - | - | - | - | - |
| Porcine bone gelatin | - | - | - | - | - | - | - | - | - | - | - | 10 | 10 | 10 | 10 | 10 | 10 | 10 | 10 | 10 | 10 | 10 |
| Calcium lactate | 5 | - | - | - | - | - | - | - | - | - | - | 5 | - | - | - | - | - | - | - | - | - | - |
| Dipotassium glycyrrhizate | - | 5 | - | - | - | - | - | - | - | - | - | - | 5 | - | - | - | - | - | - | - | - | - |
| Sodium citrate | - | - | 5 | - | - | - | - | - | - | - | - | - | - | 5 | - | - | - | - | - | - | - | - |
| Sodium gluconate | - | - | - | 5 | - | - | - | - | - | - | - | - | - | - | 5 | - | - | - | - | - | - | - |
| Sodium L-aspartate | - | - | - | - | 5 | - | - | - | - | - | - | - | - | - | - | 5 | - | - | - | - | - | - |
| Sodium malate | - | | - | - | - | 5 | - | - | - | - | - | - | - | - | - | - | 5 | - | - | - | - | - |
| Calcium gluconate | - | - | - | - | - | - | 5 | - | - | - | - | - | - | - | - | - | - | 5 | - | - | - | - |
| Disodium succinate | - | - | - | - | - | - | - | 5 | - | - | - | - | - | - | - | - | - | - | 5 | - | - | - |
| Potassium sodium tartrate | - | - | - | - | - | - | - | - | 5 | - | - | - | - | - | - | - | - | - | - | 5 | - | - |
| Sodium tartrate | - | - | - | - | - | - | - | - | - | 5 | - | - | - | - | - | - | - | - | - | - | 5 | - |
| Sodium L-ascorbate | - | - | - | - | - | - | - | - | - | - | 5 | - | - | - | - | - | - | - | - | - | - | 5 |

**[Table 8]**

| Sample | Performance | Solubility in water | | |
|---|---|---|---|---|
| | | 5 [°C] | 25 [°C] | 30 [°C] |
| Example 1 | No problem for practical use | ± | + | ++ |
| Example 2 | No problem for practical use | ± | + | ++ |
| Example 3 | No problem for practical use | ± | + | ++ |
| Example 4 | No problem for practical use | ± | + | ++ |
| Example 5 | No problem for practical use | ± | + | ++ |
| Example 6 | No problem for practical use | ± | + | ++ |
| Example 7 | No problem for practical use | ± | + | ++ |
| Example 8 | No problem for practical use | ± | + | ++ |
| Example 9 | No problem for practical use | ± | + | ++ |
| Example 10 | No problem for practical use | ± | + | ++ |
| Example 11 | No problem for practical use | ± | + | ++ |
| Example 12 | No problem for practical use | ± | + | ++ |
| Example 13 | No problem for practical use | ± | + | ++ |
| Example 14 | No problem for practical use | ± | + | ++ |
| Example 15 | No problem for practical use | ± | + | ++ |
| Example 16 | No problem for practical use | ± | + | ++ |
| Example 17 | No problem for practical use | ± | + | ++ |
| Example 18 | No problem for practical use | ± | + | ++ |
| Example 19 | No problem for practical use | ± | + | ++ |
| Example 20 | No problem for practical use | ± | + | ++ |
| Example 21 | No problem for practical use | ± | + | ++ |
| Example 22 | No problem for practical use | ± | + | ++ |

**[Table 9]**

| Sample | Remaining allergen activity | | | |
|---|---|---|---|---|
| | 30 days after | 60 days after | 90 days after | 120 days after |
| Example 1 | 5 | 5 | 5 | 5 |
| Example 2 | 5 | 5 | 5 | 5 |
| Example 3 | 5 | 5 | 5 | 5 |
| Example 4 | 5 | 5 | 5 | 5 |
| Example 5 | 5 | 5 | 5 | 5 |
| Example 6 | 5 | 5 | 5 | 5 |
| Example 7 | 5 | 5 | 5 | 5 |
| Example 8 | 5 | 5 | 5 | 4 |
| Example 9 | 5 | 5 | 5 | 4 |
| Example 10 | 5 | 5 | 5 | 5 |
| Example 11 | 5 | 5 | 5 | 4 |
| Example 12 | 5 | 5 | 5 | 5 |
| Example 13 | 5 | 5 | 5 | 5 |
| Example 14 | 5 | 5 | 5 | 5 |
| Example 15 | 5 | 5 | 5 | 5 |
| Example 16 | 5 | 5 | 5 | 5 |
| Example 17 | 5 | 5 | 5 | 5 |
| Example 18 | 5 | 5 | 5 | 5 |
| Example 19 | 5 | 5 | 5 | 4 |
| Example 20 | 5 | 5 | 5 | 4 |
| Example 21 | 5 | 5 | 5 | 5 |
| Example 22 | 5 | 5 | 5 | 4 |

Tables 8 and 9 show that all the pharmaceutical compositions prepared in Examples could be picked up with fingers without difficulty and therefore were considered to have no problem for practical use.
Regarding the solubility of the pharmaceutical compositions prepared in Examples in water, soon after addition of water, the pharmaceutical compositions broke down and lost the original shape. These pharmaceutical compositions required 1 minute or longer for complete dissolution in water at 5°C, and required about 30 seconds to 1 minute for complete dissolution in water at 25°C. The pharmaceutical compositions were completely dissolved within 30 seconds in water at 30°C.
The storage stability evaluation revealed that the allergen content did not decrease at all even after 120-days storage at 40°C.

### (Example 23)

A pharmaceutical composition was prepared by combining standardized allergen extract for subcutaneous injection "Torii" cedar pollen 2000 JAU/mL (50.0 parts by weight, from Torii Pharmaceutical Co., Ltd.) and calcium carbonate (10 parts by weight, from Wako Pure Chemical Industries, Ltd.) with water-soluble gelatin (fish origin) (CSF from Nippi Inc., 50.0 parts by weight), and agitating the mixture. This pharmaceutical composition was stored for 2 weeks at 30°C ± 2°C, and measured for remaining Cry j 1 allergen activity after the storage by the method described below.

### (Examples 24 to 31)

Pharmaceutical compositions of Examples 24 to 31 were prepared respectively using the materials shown in Table 10 in the same manner as in Example 23. These pharmaceutical compositions were stored at 30°C ± 2°C for 2 weeks and measured for remaining Cry j 1 allergen activity after the storage by the method described below.
The additives used were anhydrous calcium hydrogen phosphate (Fujicalin F from Fuji Chemical Industry Co., Ltd.), magnesium carbonate (magnesium carbonate (basic) from Nacalai Tesque), calcium silicate (from Wako Pure Chemical Industries, Ltd.), magnesium silicate (from Kyowa Chemical Industry Co., Ltd.), magnesium aluminometasilicate (Neusilin FL2 from Fuji Chemical Industry Co., Ltd.), synthetic aluminum silicate (from Kyowa Chemical Industry Co., Ltd.), sodium hydrogen phosphate (disodium hydrogen phosphate dodecahydrate from Wako Pure Chemical Industries, Ltd.), and potassium dihydrogen phosphate (from Wako Pure Chemical Industries, Ltd.), respectively.

### (Comparative Example 1)

Standardized allergen extract for subcutaneous injection "Torii" cedar pollen 2000 JAU/mL (50.0 parts by weight, from Torii Pharmaceutical Co., Ltd.) was used as a pharmaceutical composition. This pharmaceutical composition was stored at 30°C ± 2°C for 2 weeks, and measured for remaining Cry j 1 allergen activity after the storage by the method described below.

### (Comparative Example 2)

A pharmaceutical composition was prepared by combining standardized allergen extract for subcutaneous injection "Torii" cedar pollen 2000 JAU/mL (50.0 parts by weight, from Torii Pharmaceutical Co., Ltd.) with water-soluble gelatin (fish origin) (50.0 parts by weight, CSF from Nippi Inc.) and agitating the mixture. This pharmaceutical composition was stored at 30°C ± 2°C for 2 weeks, and measured for remaining Cry j 1 allergen activity after the storage by the method described below.

### (Comparative Example 3)

A pharmaceutical composition was prepared by combining standardized allergen extract for subcutaneous injection "Torii" cedar pollen 2000 JAU/mL (50.0 parts by weight, from Torii Pharmaceutical Co., Ltd.) and calcium chloride (10 parts by weight, calcium chloride dihydrate from Wako Pure Chemical Industries, Ltd.) with water-soluble gelatin (fish origin) (50.0 parts by weight, CSF from Nippi Inc.), and agitating the mixture. This pharmaceutical composition was stored at 30°C ± 2°C for 2 weeks, and measured for remaining Cry j 1 allergen activity after the storage by the method described below.

### (Comparative Examples 4 and 5)

Pharmaceutical compositions were prepared respectively using the materials shown in Table 10 in the same manner as in Comparative Example 3. These pharmaceutical compositions were stored at 30°C ± 2°C for 2 weeks, and measured for remaining Cry j 1 allergen activity after the storage by the method described below. The additives used were magnesium chloride (from Sigma) and potassium chloride (from Wako Pure Chemical Industries, Ltd.), respectively.

**[Table 10]**

| Ingredient | Amount [parts by weight] | | | | | | | | | | | | | |
|---|---|---|---|---|---|---|---|---|---|---|---|---|---|---|
| | Example | | | | | | | | | Comparative Example | | | | |
| | 23 | 24 | 25 | 26 | 27 | 28 | 29 | 30 | 31 | 1 | 2 | 3 | 4 | 5 |
| Standardized allergen extract for subcutaneous injection cedar pollen 2000 JAU/mL | 50 | 50 | 50 | 50 | 50 | 50 | 50 | 50 | 50 | 50 | 50 | 50 | 50 | 50 |
| Watersoluble gelatin (fish origin) | 50 | 50 | 50 | 50 | 50 | 50 | 50 | 50 | 50 | - | 50 | 50 | 50 | 50 |
| Calcium carbonate | 10 | - | - | - | - | - | - | - | - | - | - | - | - | - |
| Anhydrous calcium hydrogen phosphate | - | 10 | - | - | - | - | - | - | - | - | - | - | - | - |
| Magnesium carbonate | - | - | 10 | - | - | - | - | - | - | - | - | - | - | - |
| Calcium silicate | | | | 10 | - | - | | | | | | | | |
| Magnesium silicate | - | - | - | - | 10 | - | - | - | - | - | - | - | - | - |
| Magnesium aluminometasilicate | - | - | - | - | - | 10 | - | - | - | - | - | - | - | - |
| Synthetic aluminum silicate | - | - | - | - | - | - | 10 | - | - | - | - | - | - | - |
| Sodium hydrogen phosphate | - | - | - | - | - | - | - | 10 | - | - | - | - | - | - |
| Potassium dihydrogen phosphate | - | - | - | - | - | - | - | - | 10 | - | - | - | - | - |
| Calcium chloride | - | - | - | - | - | - | - | - | - | - | - | 10 | - | - |
| Magnesium chloride | - | - | - | - | - | - | - | - | - | - | - | - | 10 | - |
| Potassium chloride | - | - | - | - | - | - | - | - | - | - | - | - | - | 10 |

### (Example 32)

A pharmaceutical composition was prepared by combining standardized allergen extract for subcutaneous injection "Torii" cedar pollen 2000 JAU/mL (50.0 parts by weight, from Torii Pharmaceutical Co., Ltd.) and calcium lactate (10 parts by weight, calcium lactate hydrate (granules) from Taihei Chemical Industrial Co., Ltd.) with water-soluble gelatin (fish origin) (50.0 parts by weight, CSF from Nippi Inc.), and agitating the mixture. This pharmaceutical composition was stored at 30°C ± 2°C for 2 weeks, and measured for remaining Cry j 1 allergen activity after the storage by the method described below.

### (Examples 33 to 50)

Pharmaceutical compositions were prepared respectively using the materials shown in Table 11 in the same manner as in Example 32. These pharmaceutical compositions were stored at 30°C ± 2°C for 2 weeks, and measured for remaining Cry j 1 allergen activity after the storage by the method described below. The additives used were sodium citrate (from Wako Pure Chemical Industries, Ltd.), calcium citrate (from Wako Pure Chemical Industries, Ltd.), sodium malate (disodium DL-malate n-hydrate from Wako Pure Chemical Industries, Ltd.), dipotassium glycyrrhizate (from Wako Pure Chemical Industries, Ltd.), disodium glycyrrhizate (from Wako Pure Chemical Industries, Ltd.), calcium gluconate (calcium gluconate monohydrate from Wako Pure Chemical Industries, Ltd.), sodium gluconate (from Wako Pure Chemical Industries, Ltd.), magnesium gluconate (D-gluconic acid hemimagnesium salt from Wako Pure Chemical Industries, Ltd.), sodium stearyl fumarate (PRUV from JRS Pharma), sodium tartrate (from Wako Pure Chemical Industries, Ltd.), potassium sodium tartrate (potassium sodium tartrate tetrahydrate from Wako Pure Chemical Industries, Ltd.), disodium succinate (from Wako Pure Chemical Industries, Ltd.), sodium acetate (from Wako Pure Chemical Industries, Ltd.), sodium L-ascorbate (from Wako Pure Chemical Industries, Ltd.), sodium L-aspartate (from Wako Pure Chemical Industries, Ltd.), disodium edetate (from Wako Pure Chemical Industries, Ltd.), sodium alginate (KIMICA ALGIN from KIMICA Corp.), and sodium carboxymethylcellulose (from MP Biomedicals).

**[Table 11]**

| Ingredient | Amount [parts by weight] | | | | | | | | | | | | | | | | | | |
|---|---|---|---|---|---|---|---|---|---|---|---|---|---|---|---|---|---|---|---|
| | Example | | | | | | | | | | | | | | | | | | |
| | 32 | 33 | 34 | 35 | 36 | 37 | 38 | 39 | 40 | 41 | 42 | 43 | 44 | 45 | 46 | 47 | 48 | 49 | 50 |
| Standardized allergen extract for subcutaneous injection cedar pollen 2000 JAU/mL | 50 | 50 | 50 | 50 | 50 | 50 | 50 | 50 | 50 | 50 | 50 | 50 | 50 | 50 | 50 | 50 | 50 | 50 | 50 |
| Watersoluble gelatin (fish origin) | 50 | 50 | 50 | 50 | 50 | 50 | 50 | 50 | 50 | 50 | 50 | 50 | 50 | 50 | 50 | 50 | 50 | 50 | 50 |
| Calcium lactate | 10 | | - | - | - | - | - | - | - | - | - | - | - | - | - | - | - | - | - |
| Sodium citrate | - | 10 | - | - | - | - | - | - | - | - | - | - | - | - | - | - | - | - | - |
| Calcium citrate | - | - | 10 | - | - | - | - | - | - | - | - | - | - | - | - | - | - | - | - |
| Sodium malate | - | - | - | 10 | - | - | - | - | - | - | - | - | - | - | - | - | - | - | - |
| Dipotassium glycyrrhizate | - | - | - | - | 10 | - | - | - | - | - | - | - | - | - | - | - | - | - | - |
| Disodium glycyrrhizate | - | - | - | - | - | 10 | - | - | - | - | - | - | - | - | - | - | - | - | - |
| Calcium gluconate | - | - | - | - | - | - | 10 | - | - | - | - | - | - | - | - | - | - | - | - |
| Sodium gluconate | - | - | - | - | - | - | - | 10 | - | - | - | - | - | - | - | - | - | - | - |
| Magnesium gluconate | - | - | - | - | - | - | - | - | 10 | - | - | - | - | - | - | - | - | - | - |
| Sodium stearyl fumarate | - | - | - | - | - | - | - | - | - | 10 | - | - | - | - | - | - | - | - | - |
| Sodium tartrate | - | - | - | - | - | - | - | - | - | - | 10 | - | - | - | - | - | - | - | - |
| Potassium sodium tartrate | - | - | - | - | - | - | - | - | - | - | - | 10 | - | - | - | - | - | - | - |
| Disodium succinate | - | - | - | - | - | - | - | - | - | - | - | - | 10 | - | - | - | - | - | - |
| Sodium acetate | - | - | - | - | - | - | - | - | - | - | - | - | - | 10 | - | - | - | - | - |
| Sodium L-ascorbate | - | - | - | - | - | - | - | - | - | - | - | - | - | - | 10 | - | - | - | - |
| Sodium L-aspartate | - | - | - | - | - | - | - | - | - | - | - | - | - | - | - | 10 | - | - | - |
| Disodium edetate | - | - | - | - | - | - | - | - | - | - | - | - | - | - | - | - | 10 | - | - |
| Sodium alginate | - | - | - | - | - | - | - | - | - | - | - | - | - | - | - | - | - | 10 | - |
| Sodium carboxymethylcellulose | - | - | - | - | - | - | - | - | - | - | - | - | - | - | - | - | - | - | 10 |

### (Comparative Example 6)

A pharmaceutical composition was prepared by combining standardized allergen extract for subcutaneous injection "Torii" cedar pollen 2000 JAU/mL (50.0 parts by weight, from Torii Pharmaceutical Co., Ltd.) and lactic acid (10 parts by weight, lactic acid (Japanese Pharmacopoeia) from Komatsuya) with water-soluble gelatin (fish origin) (50.0 parts by weight, CSF from Nippi Inc.), and agitating the mixture. This pharmaceutical composition was stored at 30°C ± 2°C for 2 weeks, and measured for remaining Cry j 1 allergen activity after the storage by the method described below.

### (Comparative Examples 7 to 16)

Pharmaceutical compositions were prepared respectively using the materials shown in Table 12 in the same manner as in Comparative Example 6. These pharmaceutical compositions were stored at 30°C ± 2°C for 2 weeks, and measured for remaining Cry j 1 allergen activity after the storage by the method described below. The additives used were citric acid (citric acid hydrate (Japanese Pharmacopoeia) from Komatsuya), malic acid (DL-malic acid from Wako Pure Chemical Industries, Ltd.), glycyrrhizinic acid (from Wako Pure Chemical Industries, Ltd.), gluconic acid (50% gluconic acid solution from Wako Pure Chemical Industries, Ltd.), fumaric acid (from Wako Pure Chemical Industries, Ltd.), tartaric acid (tartaric acid (Japanese Pharmacopoeia) from Komatsuya), succinic acid (from Fuso Chemical Co., Ltd.), acetic acid (from Wako Pure Chemical Industries, Ltd.), L-ascorbic acid (from Wako Pure Chemical Industries, Ltd.), and L-asparatic acid (from Wako Pure Chemical Industries, Ltd.), respectively.

**[Table 12]**

| Ingredient | Amount [parts by weight] | | | | | | | | | | |
|---|---|---|---|---|---|---|---|---|---|---|---|
| | Comparative Example | | | | | | | | | | |
| | 6 | 7 | 8 | 9 | 10 | 11 | 12 | 13 | 14 | 15 | 16 |
| Standardized allergen extract for subcutaneous injection cedar pollen 2000 JAU/mL | 50 | 50 | 50 | 50 | 50 | 50 | 50 | 50 | 50 | 50 | 50 |
| Water-soluble gelatin (fish origin) | 50 | 50 | 50 | 50 | 50 | 50 | 50 | 50 | 50 | 50 | 50 |
| Lactic acid | 10 | - | - | - | - | - | - | - | - | - | - |
| Citric acid | - | 10 | - | - | - | - | - | - | - | - | - |
| Malic acid | - | - | 10 | - | - | - | - | - | - | - | - |
| Glycyrrhizinic acid | - | - | - | 0 | - | - | - | - | - | - | - |
| Gluconic acid | - | - | - | - | 10 | - | - | - | - | - | - |
| Fumaric acid | - | - | - | - | - | 10 | - | - | - | - | - |
| Tartaric acid | - | - | - | - | - | - | 10 | - | - | - | - |
| Succinic acid | - | - | - | - | - | - | - | 10 | - | - | - |
| Acetic acid | - | - | - | - | - | - | - | - | 10 | - | - |
| L-Ascorbic acid | - | - | - | - | - | - | - | - | - | 10 | - |
| L-Asparatic acid | - | - | - | - | - | - | - | - | - | - | 10 |

### (Example 51)

A pharmaceutical composition was prepared by agitating standardized allergen extract for subcutaneous injection "Torii" cedar pollen 2000 JAU/mL (50.0 parts by weight, from Torii Pharmaceutical Co., Ltd.) and calcium lactate (10 parts by weight, calcium lactate hydrate (granules) from Taihei Chemical Industrial Co., Ltd.) together. This pharmaceutical composition was stored at 30°C ± 2°C for 2 weeks, and measured for remaining Cry j 1 allergen activity after the storage by the method described below.

### (Examples 52 to 57)

Pharmaceutical compositions were prepared respectively using the materials shown in Table 13 in the same manner as in Example 51. These pharmaceutical compositions were stored at 30°C ± 2°C for 2 weeks, and measured for remaining Cry j 1 allergen activity after the storage by the method described below. The additives used were calcium carbonate (from Wako Pure Chemical Industries, Ltd.), magnesium carbonate (magnesium carbonate (basic) from Nacalai Tesque), magnesium silicate (from Kyowa Chemical Industry Co., Ltd.), sodium hydrogen phosphate (disodium hydrogen phosphate dodecahydrate from Wako Pure Chemical Industries, Ltd.), sodium citrate (from Wako Pure Chemical Industries, Ltd.), and sodium malate (disodium DL-malate n-hydrate from Wako Pure Chemical Industries, Ltd.), respectively.

**[Table 13]**

| Ingredient | Amount [parts by weight] | | | | | | |
|---|---|---|---|---|---|---|---|
| | Example | | | | | | |
| | 51 | 52 | 53 | 54 | 55 | 56 | 57 |
| Standardized allergen extract for subcutaneous injection cedar pollen 2000 JAU/mL | 50 | 50 | 50 | 50 | 50 | 50 | 50 |
| Calcium lactate | 10 | - | - | - | - | - | - |
| Calcium carbonate | - | 10 | - | - | - | - | - |
| Magnesium carbonate | - | - | 10 | - | - | - | - |
| Magnesium silicate | - | - | - | 10 | - | - | - |
| Sodium hydrogen phosphate | - | - | - | - | 10 | - | - |
| Sodium citrate | - | - | - | - | - | 10 | - |
| Sodium malate | - | - | - | - | - | - | 10 |

### (Example 58)

Pure water (25.0 parts by weight) was added to water-soluble gelatin (fish origin) (50.0 parts by weight from CSF, Nippi Inc.). Then, pure cedar pollen antigen Cry j 1 (0.05 parts by weight, from Seikagaku Biobusiness Corp.) and calcium lactate (10 parts by weight, calcium lactate hydrate (granules) from Taihei Chemical Industrial Co., Ltd.) were added thereto, and the mixture was agitated. This mixture was stored at 30°C ± 2°C for 2 weeks, and measured for remaining Cry j 1 allergen activity after the storage.

### (Examples 59 to 64)

Mixtures were prepared respectively using the materials shown in Table 14 in the same manner as in Example 58. These mixtures were stored at 30°C ± 2°C for 2 weeks, and measured for remaining Cry j 1 allergen activity after the storage. The additives used were calcium carbonate (from Wako Pure Chemical Industries, Ltd.), magnesium carbonate (magnesium carbonate (basic) from Nacalai Tesque), magnesium silicate (from Kyowa Chemical Industry Co., Ltd.), sodium hydrogen phosphate (disodium hydrogen phosphate dodecahydrate from Wako Pure Chemical Industries, Ltd.), sodium citrate (from Wako Pure Chemical Industries, Ltd.), and sodium malate (disodium DL-malate n-hydrate from Wako Pure Chemical Industries, Ltd.), respectively.

### (Comparative Example 17)

A pharmaceutical composition was prepared by adding pure water (25.0 parts by weight) to water-soluble gelatin (fish origin) (50.0 parts by weight, CSF from Nippi Inc.), further adding pure cedar pollen antigen Cry j 1 (0.05 parts by weight, from Seikagaku Biobusiness Corp.) thereto, and agitating the mixture. This pharmaceutical composition was stored at 30°C ± 2°C for 2 weeks, and measured for remaining Cry j 1 allergen activity after the storage by the method described below.

### (Comparative Example 18)

A pharmaceutical composition was prepared by adding pure water (25.0 parts by weight) to pure cedar pollen antigen Cry j 1 (0.05 parts by weight, from Seikagaku Biobusiness Corp.), and agitating the mixture. This pharmaceutical composition was stored at 30°C ± 2°C for 2 weeks, and measured for remaining Cry j 1 allergen activity after the storage by the method described below.

**[Table 14]**

| Ingredient | Amount [parts by weight] | | | | | | | | |
|---|---|---|---|---|---|---|---|---|---|
| | Example | | | | | | | Comparative Example | |
| | 58 | 59 | 60 | 61 | 62 | 63 | 64 | 17 | 18 |
| Pure cedar pollen antigen Cryj1 | 0.05 | 0.05 | 0.05 | 0.05 | 0.05 | 0.05 | 0.05 | 0.05 | 0.05 |
| Water-soluble gelatin (fish origin) | 50 | 50 | 50 | 50 | 50 | 50 | 50 | 50 | - |
| Calcium lactate | 10 | - | - | - | - | - | - | - | - |
| Calcium carbonate | - | 10 | - | - | - | - | - | - | - |
| Magnesium carbonate | - | - | 10 | - | - | - | - | - | - |
| Magnesium silicate | - | - | - | 10 | - | - | - | - | - |
| Sodium hydrogen phosphate | - | - | - | - | 10 | - | - | - | - |
| Sodium citrate | - | - | - | - | - | 10 | - | - | - |
| Sodium malate | - | - | - | - | - | - | 10 | - | - |
| Pure water | 25 | 25 | 25 | 25 | 25 | 25 | 25 | 25 | 25 |

### (Example 65)

A pharmaceutical composition was prepared by adding pure water (25.0 parts by weight) to water-soluble gelatin (fish origin) (50.0 parts by weight, CSF from Nippi Inc.), further adding cedar pollen extract lyophilized powder (0.1 parts by weight, from LSL Co., Ltd.), calcium lactate (10 parts by weight, calcium lactate hydrate (granules) from Taihei Chemical Industrial Co., Ltd.) thereto, and agitating the mixture. This pharmaceutical composition was stored at 30°C ± 2°C for 2 weeks, and measured for remaining Cry j 1 allergen activity after the storage by the method described below.

### (Examples 66 to 71)

Pharmaceutical compositions were prepared respectively using the materials shown in Table 15 in the same manner as in Example 65. These pharmaceutical compositions were stored at 30°C ± 2°C for 2 weeks, and measured for remaining Cry j 1 allergen activity after the storage by the method described below. The additives used were calcium carbonate (from Wako Pure Chemical Industries, Ltd.), magnesium carbonate (magnesium carbonate (basic) from Nacalai Tesque), magnesium silicate (from Kyowa Chemical Industry Co., Ltd.), sodium hydrogen phosphate (disodium hydrogen phosphate dodecahydrate from Wako Pure Chemical Industries, Ltd.), sodium citrate (from Wako Pure Chemical Industries, Ltd.), and sodium malate (disodium DL-malate n-hydrate from Wako Pure Chemical Industries, Ltd.), respectively.

### (Comparative Example 19)

A pharmaceutical composition was prepared by adding pure water (25.0 parts by weight) to water-soluble gelatin (fish origin) (50.0 parts by weight, CSF from Nippi Inc.), further adding cedar pollen extract lyophilized powder (0.1 parts by weight, from LSL Co., Ltd.) thereto, and agitating the mixture. This pharmaceutical composition was stored at 30°C ± 2°C for 2 weeks, and measured for remaining Cry j 1 allergen activity after the storage by the method described below.

### (Comparative Example 20)

A pharmaceutical composition was prepared by adding pure water (25.0 parts by weight) to cedar pollen extract lyophilized powder (0.1 parts by weight, from LSL Co., Ltd.), and agitating the mixture. This pharmaceutical composition was stored at 30°C ± 2°C for 2 weeks, and measured for remaining Cry j 1 allergen activity after the storage by the method described below.

**[Table 15]**

| Ingredient | Amount [parts by weight] | | | | | | | | |
|---|---|---|---|---|---|---|---|---|---|
| | Example | | | | | | | Comparative Example | |
| | 65 | 66 | 67 | 68 | 69 | 70 | 71 | 19 | 20 |
| Cedar pollen extract lyophilized powder | 0.1 | 0.1 | 0.1 | 0.1 | 0.1 | 0.1 | 0.1 | 0.1 | 0.1 |
| Water-soluble gelatin (fish origin) | 50 | 50 | 50 | 50 | 50 | 50 | 50 | 50 | - |
| Calcium lactate | 10 | - | - | - | - | - | - | - | - |
| Calcium carbonate | - | 10 | - | - | - | - | - | - | - |
| Magnesium carbonate | - | - | 10 | - | - | - | - | - | - |
| Magnesium silicate | - | - | - | 10 | - | - | - | - | - |
| Sodium hydrogen phosphate | - | - | - | - | 10 | - | - | - | - |
| Sodium citrate | - | - | - | - | - | 10 | - | - | - |
| Sodium malate | - | - | - | - | - | - | 10 | - | - |
| Pure water | 25 | 25 | 25 | 25 | 25 | 25 | 25 | 25 | 25 |

### (Example 72)

Standardized allergen extract for subcutaneous injection "Torii" cedar pollen 2000 JAU/mL (50.0 parts by weight, from Torii Pharmaceutical Co., Ltd.) and citric acid (0.1 parts by weight, citric acid (citric acid hydrate (Japanese Pharmacopoeia) from Komatsuya) were combined with water-soluble gelatin (fish origin) (50.0 parts by weight, CSF from Nippi Inc.), and the resulting mixture was agitated. This mixture was stored at 30°C ± 2°C for 2 weeks, and measured for remaining Cry j 1 allergen activity after the storage by the method described below. Since the standardized allergen extract for subcutaneous injection "Torii" cedar pollen 2000 JAU/mL (from Torii Pharmaceutical Co., Ltd.) contains NaCl, sodium citrate was present in the resulting mixture.

### (Examples 73 to 75)

Pharmaceutical compositions were prepared respectively using the materials shown in Table 16 in the same manner as in Example 72. These pharmaceutical compositions were stored at 30°C ± 2°C for 2 weeks, and measured for remaining Cry j 1 allergen activity after the storage by the method described below.

**[Table 16]**

| Ingredient | Amount [parts by weight] | | | |
|---|---|---|---|---|
| | Example | | | |
| | 72 | 73 | 74 | 75 |
| Standardized allergen extract for subcutaneous injection cedar pollen 2000 JAU/mL | 50 | 50 | 50 | 50 |
| Water-soluble gelatin (fish origin) | 50 | 50 | 50 | 50 |
| Citric acid | 0.1 | 0.2 | 0.5 | 1.0 |

### (Example 76)

Polysorbate-80 (0.1 parts by weight, NIKKOL TO-10MV from Nikko Chemicals Co., Ltd.), medium-chain triglyceride (0.1 parts by weight, COCONAR MT from Kao Corp.), and methyl p-hydroxybenzoate (0.1 parts by weight, methyl p-hydroxybenzoate from Ueno Fine Chemicals Industry) were added to pure water (20.0 parts by weight) and dissolved therein by 10-minute ultrasonic agitation. Water-soluble gelatin (fish origin) (12.0 parts by weight, CSF from Nippi Inc.) was added to the solution, and the mixture was agitated at 35°C for 30 minutes. In this manner, a gelatin solution was prepared. Separately, standardized allergen extract for subcutaneous injection "Torii" cedar pollen 2000 JAU/mL (55.0 parts by weight, from Torii Pharmaceutical Co., Ltd.) was combined with D-sorbitol (8.0 parts by weight, NEOSORB P60W from ROQUETTE), calcium carbonate (3.0 parts by weight, from Wako Pure Chemical Industries, Ltd.), sodium citrate (1.0 part by weight, from Wako Pure Chemical Industries, Ltd.), and aspartame (0.1 parts by weight, Ajinomoto KK aspartame from Ajinomoto Co., Inc.), and the mixture was agitated. In this manner, a pharmaceutical composition was prepared. The whole pharmaceutical composition was combined with the gelatin solution prepared above, and a cherry flavor (0.1 parts by weight, from Takasago International Corp.) was added thereto. The mixture was agitated at 35°C for 20 minutes, and then, a 2.0-g portion thereof was dispensed to a 5-cm² plastic blister case (Cryomold (square type) No. 3 from Sakura Finetek Japan Co., Ltd.), and cool-solidified at 2°C to 8°C overnight. In this manner, a jelly preparation was prepared. This jelly preparation was stored at 30°C ± 2°C for 3 months, and measured for remaining Cry j 1 allergen activity after the storage by the method described below.

### (Examples 77 and 78)

Jelly preparations were prepared respectively using the materials shown in Table 17 in the same manner as in Example 76.
These jelly preparations were stored at 30°C ± 2°C for 3 months, and measured for remaining Cry j 1 allergen activity after the storage by the method described below. The additive used was calcium lactate (calcium lactate hydrate (granules) from Taihei Chemical Industrial Co., Ltd.).

### (Example 79)

Polysorbate-80 (0.1 parts by weight, NIKKOL TO-10MV from Nikko Chemicals Co., Ltd.), medium-chain triglyceride (0.1 parts by weight, COCONAR MT from Kao Corp.), and methyl p-hydroxybenzoate (0.1 parts by weight, methyl p-hydroxybenzoate from Ueno Fine Chemicals Industry) were added to pure water (20.0 parts by weight), and dissolved therein by 10-minute ultrasonic agitation. Water-soluble gelatin (fish origin) (12.0 parts by weight, CSF from Nippi Inc.) was added to the solution, and the mixture was agitated at 35°C for 30 minutes. In this manner, a gelatin solution was prepared. Separately, standardized allergen extract for subcutaneous injection "Torii" cedar pollen 2000 JAU/mL (55.0 parts by weight, from Torii Pharmaceutical Co., Ltd.) was combined with D-sorbitol (8.0 parts by weight, NEOSORB P60W from ROQUETTE), calcium carbonate (3.0 parts by weight, from Wako Pure Chemical Industries, Ltd.), sodium citrate (1.0 part by weight, from Wako Pure Chemical Industries, Ltd.), and aspartame (0.1 parts by weight, Ajinomoto KK aspartame from Ajinomoto Co., Inc.), and the mixture was agitated. In this manner, a pharmaceutical composition was prepared. The whole pharmaceutical composition was combined with the gelatin solution prepared above, and a cherry flavor (0.1 parts by weight, from Takasago International Corp.) was added thereto. The mixture was agitated at 35°C for 20 minutes, and then adjusted to pH 7.0 with phosphoric acid (from Wako Pure Chemical Industries, Ltd.), and further agitated for 5 minutes. Subsequently, a 2.0-g portion thereof was dispensed to a 5-cm² plastic blister case (Cryomold (square type) No. 3 from Sakura Finetek Japan Co., Ltd.), and cool-solidified at 2°C to 8°C overnight. In this manner, a jelly preparation was prepared. This jelly preparation was stored at 30°C ± 2°C for 3 months, and measured for remaining Cry j 1 allergen activity after the storage by the method described below.

### (Examples 80 and 81)

Jelly preparations were prepared respectively using the materials shown in Table 17 in the same manner as in Example 79.
These jelly preparations were stored at 30°C ± 2°C for 3 months, and measured for remaining Cry j 1 allergen activity after the storage by the method described below.

**[Table 17]**

| Ingredient | Amount [parts by weight] | | | | | |
|---|---|---|---|---|---|---|
| | Example | | | | | |
| | 76 | 77 | 78 | 79 | 80 | 81 |
| Standardized allergen extract for subcutaneous injection cedar pollen 2000 JAU/mL | 55.0 | 55.0 | 55.0 | 55.0 | 55.0 | 55.0 |
| Water-soluble gelatin (fish origin) | 12.0 | 12.0 | 12.0 | 12.0 | 12.0 | 12.0 |
| Calcium carbonate | 3.0 | - | - | 3.0 | 3.0 | - |
| Calcium lactate | - | 3.0 | - | - | - | - |
| Sodium citrate | 1.0 | 1.0 | 1.0 | 1.0 | - | 1.0 |
| Phosphoric acid | - | - | - | Adequate amount | Adequate amount | Adequate amount |
| D-Sorbitol | 8.0 | 8.0 | 8.0 | 8.0 | 8.0 | 8.0 |
| Polysorbate-80 | 0.1 | 0.1 | 0.1 | 0.1 | 0.1 | 0.1 |
| Medum-chain triglyceride | 0.1 | 0.1 | 0.1 | 0.1 | 0.1 | 0.1 |
| Aspartame | 0.1 | 0.1 | 0.1 | 0.1 | 0.1 | 0.1 |
| Cherry flavor | 0.1 | 0.1 | 0.1 | 0.1 | 0.1 | 0.1 |
| Methyl p-hydroxybenzoate | 0.1 | 0.1 | 0.1 | 0.1 | 0.1 | 0.1 |
| Pure water | 20.0 | 20.0 | 23.0 | 20.0 | 21.0 | 23.0 |

### (Test method)

Whether the pharmaceutical compositions contribute to the stability (in particular, heat stability) of the cedar pollen allergen protein Cry j 1 was evaluated by measuring the remaining allergen activity of the Cry j 1 protein after 2-week storage by the method described below. Tables 18 to 25 show the results.

### (Evaluation method for remaining Cry j 1 allergen activity)

The allergen activity of Cry j 1, one of the major allergens of cedar pollens, was measured using a cedar pollen antigen ELISA Kit "Cry j1" (from Seikagaku Biobusiness Corp.). The principle of the measurement kit is a sandwich ELISA method that utilizes monoclonal antibodies (013, 053) specific to Cry j 1, which is one of the Japanese Cedar (Cryptomeria japonica) pollen antigens, and the method allows specific Cry j 1 measurement.
To 100 µL of a reaction buffer solution included in the kit was added 20 µL of a standard solution or sample, and a primary reaction was carried out at ambient temperature for 60 minutes. Then, 100 µL of an HRP-labeled antibody solution was added thereto and a secondary reaction was carried out for 60 minutes. Added thereto was 100 µL of an enzyme substrate solution, and a reaction was carried out for 30 minutes at ambient temperature while light was shielded. Finally, 100 µL of a reaction stop solution was added thereto. Thereafter, the ultraviolet absorption intensity at 450 nm was measured. A calibration curve was determined based on the absorption intensity of the standard solution at various Cry j 1 concentrations, and the Cry j 1 allergen activity (ng/mL) of each sample was determined based on the calibration curve.
The initial activity of the Cry j 1 to each sample was taken as 100%, and the remaining Cry j 1 allergen. activity (%) after storage relative to the initial activity was determined. The remaining Cry j 1 allergen activity (%) was evaluated based on the following scoring criteria.
5: not less than 90% and less than 105%
4: not less than 75% and less than 90%
3: not less than 60% and less than 75%
2: not less than 45% and less than 60%
1: less than 45%

**[Table 18]**

| Sample | Remaining Cryj1 allergen activity |
|---|---|
| | 2 weeks after |
| Example 23 | 5 |
| Example 24 | 3 |
| Example 25 | 5 |
| Example 26 | 5 |
| Example 27 | 5 |
| Example 28 | 4 |
| Example 29 | 4 |
| Example 30 | 5 |
| Example 31 | 3 |
| Comparative Example 1 | 1 |
| Comparative Example 2 | 2 |
| Comparative Example 3 | 1 |
| Comparative Example 4 | 1 |
| Comparative Example 5 | 1 |

**[Table 19]**

| Sample | Remaining Cryj1 allergen activity |
|---|---|
| | 2 weeks after |
| Example 32 | 5 |
| Example 33 | 4 |
| Example 34 | 4 |
| Example 35 | 4 |
| Example 36 | 4 |
| Example 37 | 3 |
| Example 38 | 4 |
| Example 39 | 4 |
| Example 40 | 4 |
| Example 41 | 3 |
| Example 42 | 4 |
| Example 43 | 4 |
| Example 44 | 3 |
| Example 45 | 4 |
| Example 46 | 3 |
| Example 47 | 3 |
| Example 48 | 3 |
| Example 49 | 3 |
| Example 50 | 4 |

**[Table 20]**

| Sample | Remaining Cryj1 allergen activity |
|---|---|
| | 2 weeks after |
| Comparative Example 6 | 1 |
| Comparative Example 7 | 1 |
| Comparative Example 8 | 1 |
| Comparative Example 9 | 1 |
| Comparative Example 10 | 1 |
| Comparative Example 11 | 1 |
| Comparative Example 12 | 1 |
| Comparative Example 13 | 1 |
| Comparative Example 14 | 1 |
| Comparative Example 15 | 1 |
| Comparative Example 16 | 1 |

**[Table 21]**

| Sample | Remaining Cryj1 allergen activity |
|---|---|
| | 2 weeks after |
| Example 51 | 5 |
| Example 52 | 4 |
| Example 53 | 4 |
| Example 54 | 4 |
| Example 55 | 5 |
| Example 56 | 4 |
| Example 57 | 3 |

**[Table 22]**

| Sample | Remaining Cryj1 allergen activity |
|---|---|
| | 2 weeks after |
| Example 58 | 5 |
| Example 59 | 4 |
| Example 60 | 4 |
| Example 61 | 4 |
| Example 62 | 5 |
| Example 63 | 4 |
| Example 64 | 3 |
| Comparative Example 17 | 2 |
| Comparative Example 18 | 1 |

**[Table 23]**

| Sample | Remaining Cryj1 allergen activity |
|---|---|
| | 2 weeks after |
| Example 65 | 5 |
| Example 66 | 5 |
| Example 67 | 4 |
| Example 68 | 5 |
| Example 69 | 5 |
| Example 70 | 4 |
| Example 71 | 3 |
| Comparative Example 19 | 2 |
| Comparative Example 20 | 1 |

**[Table 24]**

| Sample | Remaining Cryj1 allergen activity |
|---|---|
| | 2 weeks after |
| Example 72 | 3 |
| Example 73 | 4 |
| Example 74 | 4 |
| Example 75 | 2 |

**[Table 25]**

| Sample | Remaining Cryj1 allergen activity |
|---|---|
| | 3 months after |
| Example 76 | 5 |
| Example 77 | 5 |
| Example 78 | 4 |
| Example 79 | 5 |
| Example 80 | 5 |
| Example 81 | 5 |

Tables 18 to 25 show that the pharmaceutical compositions and jelly preparations of Examples each of which contained the allergen and the organic acid salt and/or the inorganic acid salt were given good remaining Cry j 1 allergen activity scores. The reason why the pharmaceutical composition of Example 59 was given a slightly low remaining Cry j 1 allergen activity score is presumably that since the amount of citric acid added was too large and larger than the amount of sodium chloride in the standardized allergen extract for subcutaneous injection "Torii" cedar pollen 2000 JAU/mL, the labilization effect of the citric acid was stronger than the stabilization effect of sodium citrate produced in the pharmaceutical composition.
In contrast, all of the pharmaceutical compositions of Comparative Examples were given a remaining Cry j 1 allergen activity score of 1 or 2.

### (Experimental Examples 32 to 34)

Sodium carbonate (from Wako Pure Chemical Industries, Ltd.) was added in an amount (mg) shown in Table 26 to standardized allergen extract for subcutaneous injection "Torii" cedar pollen 200 JAU/mL (1.0 mL, from Torii Pharmaceutical Co., Ltd.), and the mixture was agitated. In this manner, pharmaceutical compositions were prepared respectively. These pharmaceutical compositions were stored at 40°C ± 2°C for 2 weeks, and measured for remaining Cry j 1 allergen activity after the storage by the method described below.

### (Comparative Experimental Example 9)

Standardized allergen extract for subcutaneous injection "Torii" cedar pollen 200 JAU/mL (1.0 mL from Torii Pharmaceutical Co., Ltd.) was used as a pharmaceutical composition. This pharmaceutical composition was stored at 40°C ± 2°C for 2 weeks, and measured for remaining Cry j 1 allergen activity after the storage by the method described below.

### (Comparative Experimental Examples 10 and 11)

Sodium carbonate (from Wako Pure Chemical Industries, Ltd.) was added in an amount (mg) shown in Table 26 to standardized allergen extract for subcutaneous injection "Torii" cedar pollen 200 JAU/mL (1.0 mL, from Torii Pharmaceutical Co., Ltd.), and the mixture was agitated. In this manner, pharmaceutical compositions were prepared respectively. These pharmaceutical compositions were stored at 40°C ± 2°C for 2 weeks, and measured for remaining Cry j 1 allergen activity after the storage by the method described below.

**[Table 26]**

| Ingredient | Amount | | | | | |
|---|---|---|---|---|---|---|
| | Experimental Example | | | Comparative Experimental Example | | |
| | 32 | 33 | 34 | 9 | 10 | 11 |
| Standardized allergen extract for subcutaneous injection cedar pollen 200 JAU/mL [mL] | 1.0 | 1.0 | 1.0 | 1.0 | 1.0 | 1.0 |
| Sodium carbonate [mg] | 0.01 | 0.05 | 0.08 | - | 0.13 | 0.77 |

### (Experimental Examples 35 to 37)

Sodium carbonate (from Wako Pure Chemical Industries, Ltd.) was added in an amount (mg) shown in Table 27 to standardized allergen extract for subcutaneous injection "Torii" cedar pollen 2000 JAU/mL (1.0 mL, from Torii Pharmaceutical Co., Ltd.), and the mixture was agitated. In this manner, pharmaceutical compositions were prepared respectively. These pharmaceutical compositions were stored at 40°C ± 2°C for 2 weeks, and measured for remaining Cry j 1 allergen activity after the storage by the method described below.

### (Comparative Experimental Example 12)

Standardized allergen extract for subcutaneous injection "Torii" cedar pollen 2000 JAU/mL (1.0 mL, from Torii Pharmaceutical Co., Ltd.) was used as a pharmaceutical composition. This pharmaceutical composition was stored at 40°C ± 2°C for 2 weeks, and measured for remaining Cry j 1 allergen activity after the storage by the method described below.

### (Comparative Experimental Examples 13 to 15)

Sodium carbonate (from Wako Pure Chemical Industries, Ltd.) was added in an amount (mg) shown in Table 27 to standardized allergen extract for subcutaneous injection "Torii" cedar pollen 2000 JAU/mL (1.0 mL, from Torii Pharmaceutical Co., Ltd.), and the mixture was agitated. In this manner, pharmaceutical compositions were prepared respectively. These pharmaceutical compositions were stored at 40°C ± 2°C for 2 weeks, and measured for remaining Cry j 1 allergen activity after the storage by the method described below.

**[Table 27]**

| Ingredient | Amount | | | | | | |
|---|---|---|---|---|---|---|---|
| | Experimental Example | | | Comparative Experimental Example | | | |
| | 35 | 36 | 37 | 12 | 13 | 14 | 15 |
| Standardized allergen extract for subcutaneous injection cedar pollen 2000 JAU/mL [mL] | 1.0 | 1.0 | 1.0 | 1.0 | 1.0 | 1.0 | 1.0 |
| Sodium carbonate [mg] | 0.05 | 0.11 | 0.17 | - | 0.01 | 0.26 | 0.87 |

### (Experimental Examples 76 to 80 and Comparative Experimental Examples 16 and 17)

A 80 mM Britton-Robinson buffer (BR Buffer) was prepared using acetic acid, phosphoric acid, and boric acid (all available from Wako Pure Chemical Industries, Ltd.). The obtained BR Buffer was adjusted to a pH shown in Table 28 with a sodium hydroxide solution (from Wako Pure Chemical Industries, Ltd.).
Then, an allergen solution was prepared by adding pure water (2.9 mL) to cedar pollen extract lyophilized powder (2 mg, cedar pollen extract-Cj from LSL Co., Ltd.) and dissolving the powder enough. According to the attached document, the obtained allergen solution was a 5-mM boric acid buffer (pH = 8.0). Glycerin (250.0 parts by weight) was added to a 125.0 parts by weight portion of the allergen solution, and the resulting mixture was agitated. The BR Buffer (125.0 parts by weight) adjusted to a pH shown in Table 28 was added to the mixture, and the mixture was agitated enough. In this manner, pharmaceutical compositions were prepared respectively. These pharmaceutical compositions were stored at 40°C ± 2°C for 2 weeks, and measured for remaining Cry j 1 allergen activity after the storage by the method described below.

**[Table 28]**

| Ingredient | Amount [parts by weight] | | | | | | |
|---|---|---|---|---|---|---|---|
| | Experimental Example | | | | | Comparative Experimental Example | |
| | 38 | 39 | 40 | 41 | 42 | 16 | 17 |
| Cedar pollen extract-Cj 5-mM boric acid buffer | 125.0 | 125.0 | 125.0 | 125.0 | 125.0 | 125.0 | 125.0 |
| 80 mM BR Buffer [pH = 8.0] | 125.0 | - | - | - | - | - | - |
| 80 mM BR Buffer [pH = 9.0] | - | 25.0 | - | - | - | - | - |
| 80 mM BR Buffer [pH = 10.0] | - | - | 125.0 | - | - | - | - |
| 80 mM BR Buffer [pH = 10.7] | - | - | - | 125.0 | - | - | - |
| 80 mM BR Buffer [pH = 4.0] | - | - | - | - | - | 125.0 | - |
| 80 mM BR Buffer [pH = 6.0] | - | - | - | - | 125.0 | - | - |
| 80 mM BR Buffer [pH = 11.0] | - | - | - | - | - | - | 125.0 |
| Glycerin | 250.0 | 250.0 | 250.0 | 250.0 | 250.0 | 250.0 | 250.0 |

### (Experimental Examples 43 to 46 and Comparative Experimental Examples 18 to 21)

Polysorbate-80 as an antifoamer, medium-chain triglyceride (0.1 parts by weight, CCTG), and methyl p-hydroxybenzoate (0.1 parts by weight, methylparaben) as an antiseptic were added to pure water in amounts shown in Table 29, and dissolved and dispersed by ultrasonic waves. Sodium carbonate in an amount shown in Table 29 was added and dissolved in this mixture.
Subsequently, a gelatin solution was prepared by dissolving water-soluble fish gelatin (10 parts by weight, CSF from Nippi Inc.) in the mixture at 30°C to 40°C, and agitating the resulting mixture on a shaker at a constant temperature of 28°C to 32°C. Separately, a 50 parts by weight portion of the cedar pollen allergen extract stock solution 2000 JAU/mL was sampled, and D-sorbitol (10 parts by weight) was dissolved therein at 2°C to 8°C. The resulting solution was heated to 25°C to 30°C. Then, the whole gelatin solution prepared above was added to this solution, and the mixture was immediately agitated at 28°C to 32°C. Then, a 1-g portion of the mixture was dispensed to a 5-cm² plastic blister case (Cryomold (square type) No. 3 from Sakura Finetek Japan Co., Ltd.), and cool-solidified at 2°C to 8°C overnight. In this manner, jelly preparations were prepared respectively. These jelly preparations were stored at 25°C ± 2°C for 2 months, and measured for remaining Cry j 1 allergen activity after the storage by the method described below.

**[Table 29]**

| Ingredient | Experimental Example [parts by weight] | | | | Comparative Experimental Example [parts by weight] | | | |
|---|---|---|---|---|---|---|---|---|
| | 43 | 44 | 45 | 46 | 18 | 19 | 20 | 21 |
| Standardized allergen extract for subcutaneous injection cedar pollen 2000 JAU/mL | 50.0 | 50.0 | 50.0 | 50.0 | 50.0 | 50.0 | 50.0 | 50.0 |
| Water-soluble fish gelatin | 10.0 | 10.0 | 10.0 | 10.0 | 10.0 | 10.0 | 10.0 | 10.0 |
| D-Sorbitol | 10.0 | 10.0 | 10.0 | 10.0 | 10.0 | 10.0 | 10.0 | 10.0 |
| Polysorbate-80 | 0.1 | 0.1 | 0.1 | 0.1 | 0.1 | 0.1 | 0.1 | 0.1 |
| Medium-chain triglyceride | 0.1 | 0.1 | 0.1 | 0.1 | 0.1 | 0.1 | 0.1 | 0.1 |
| Methyl p-hydroxybenzoate | 0.1 | 0.1 | 0.1 | 0.1 | 0.1 | 0.1 | 0.1 | 0.1 |
| Sodium carbonate | 0.08 | 0.16 | 0.27 | 0.36 | 0.00 | 0.04 | 0.49 | 1.09 |
| *Pure water* | *39.62* | *39.54* | *39.43* | *39.34* | *39.70* | *39.66* | *39.21* | *38.61* |
| Dispensed amount [g/blister] | 1 | 1 | 1 | 1 | 1 | 1 | 1 | 1 |
| Size [cm²] | 5 | 5 | 5 | 5 | 5 | 5 | 5 | 5 |

### (Comparative Experimental Example 22)

Standardized allergen extract for subcutaneous injection "Torii" cedar pollen 2000 JAU/mL (50.0 parts by weight, from Torii Pharmaceutical Co., Ltd.) was used as a pharmaceutical composition. This pharmaceutical composition was stored at 25°C ± 2°C for 2 months, and measured for remaining Cry j 1 allergen activity after the storage by the method described below.

### (Experimental Examples 47 to 50, Comparative Experimental Examples 23 and 24)

Jelly preparations were prepared respectively in the same manner as in Experimental Example 43, except that the materials shown in Table 30 were used. These jelly preparations were stored at 25°C ± 2°C for 2 months, and measured for remaining Cry j 1 allergen activity after the storage by the method described below.
Porcine gelatin (AEP from Nippi Inc.) was used in Experimental Examples 47 to 50 and Comparative Experimental Examples 23 and 24.

**[Table 30]**

| Ingredient | Experimental Example [parts by weight] | | | | Comparative Experimental Example [parts by weight] | | |
|---|---|---|---|---|---|---|---|
| | 47 | 48 | 49 | 50 | 22 | 23 | 24 |
| Standardized allergen extract for subcutaneous injection cedar pollen 2000 JAU/mL | 50.0 | 50.0 | 50.0 | 50.0 | 50.0 | 50.0 | 50.0 |
| Porcine gelatin | 10.0 | 10.0 | 10.0 | 10.0 | - | 10.0 | 10.0 |
| D-Sorbitol | 10.0 | 10.0 | 10.0 | 10.0 | - | 10.0 | 10.0 |
| Polysorbate-80 | 0.1 | 0.1 | 0.1 | 0.1 | - | 0.1 | 0.1 |
| Medium-chain triglyceride | 0.1 | 0.1 | 0.1 | 0.1 | - | 0.1 | 0.1 |
| Methyl p-hydroxybenzoate | 0.1 | 0.1 | 0.1 | 0.1 | - | 0.1 | 0.1 |
| Sodium carbonate | 0.00 | 0.02 | 0.07 | 0.14 | - | 0.24 | 0.79 |
| Pure water | 39.70 | 39.68 | 39.63 | 39.56 | - | 39.46 | 38.91 |
| Dispensed amount [g/blister] | 1 | 1 | 1 | 1 | - | 1 | 1 |
| Size [cm²] | 5 | 5 | 5 | 5 | - | 5 | 5 |

### (Test method)

The pH of the pharmaceutical compositions and jelly preparations prepared in Experimental Examples and Comparative Experimental Examples was measured.
Whether the pharmaceutical compositions contribute to the stability (in particular, heat stability) of the cedar pollen allergen protein Cry j 1 was evaluated by measuring the allergen activity of the Cry j 1 protein. The test methods are described below, and the results are shown in Tables 31 to 33.

### (pH measurement method)

The prepared pharmaceutical compositions (Experimental Examples 32 to 42 and Comparative Experimental Examples 9 to 17) were measured for pH using a pH meter (pH meter from HORIBA Ltd.) at 25°C ± 2°C.
Also, a 1-g sample of each jelly preparation (Experimental Examples 43 to 50 and Comparative Experimental Examples 18 to 21, 23, and 24) or a 0.5-mL portion of the pharmaceutical composition (Comparative Experimental Example 22) was fed to a 10-mL graduated flask, and diluted with distilled water. In the case of the jelly preparations, a sample solution of each preparation was obtained by completely dissolving the preparation by agitation at a constant temperature of 30°C to 35°C. Then, each sample solution was measured for pH using the pH meter (pH meter from HORIBA Ltd.) at a temperature of 25°C ± 2°C.

### (Allergen activity evaluation method)

The allergen activity of Cry j 1, one of the major allergens of cedar pollens, was measured using a cedar pollen antigen ELISA Kit "Cry j1" (from Seikagaku Biobusiness Corp.). The principle of the measurement kit is a sandwich ELISA method that utilizes monoclonal antibodies (013, 053) specific to Cry j 1, which is one of the Japanese Cedar (Cryptomeria japonica) pollen antigens, and the method allows specific Cry j 1 measurement. To 100 µL of a reaction buffer solution included in the kit was added 20 µL of a standard solution or sample, and a primary reaction was carried out at ambient temperature for 60 minutes. Then, 100 µL of an HRP-labeled antibody solution was added thereto and a secondary reaction was carried out for 60 minutes. Added thereto was 100 µL of an enzyme substrate solution, and a reaction was carried out for 30 minutes at ambient temperature while light was shielded. Finally, 100 µL of a reaction stop solution was added thereto. Thereafter, the ultraviolet absorption intensity at 450 nm was measured. A calibration curve was determined based on the absorption intensity of the standard solution at various Cry j 1 concentrations, and the Cry j 1 allergen activity (ng/mL) of each sample was determined based on the calibration curve.
The initial activity of the Cry j 1 to each sample was taken as 100%, and the Cry j 1 allergen activity (%) relative to the initial activity was determined after 1 day, 7 days, and 14 days. The Cry j 1 allergen activity (%) was evaluated based on the following scoring criteria.
5: not less than 90% and less than 105%
4: not less than 75% and less than 90%
3: not less than 60% and less than 75%
2: not less than 45% and less than 60%
1: not less than 30% and less than 45%
0: less than 30%

**[Table 31]**

| | pH | Remaining Cryj1 allergen activity | | |
|---|---|---|---|---|
| | | 1 day after | 7 days after | 14 days after |
| Experimental Example 32 | 6.4 | 4 | 3 | 3 |
| Experimental Example 33 | 7.5 | 4 | 3 | 3 |
| Experimental Example 34 | 8.4 | 4 | 2 | 2 |
| Comparative Experimental Example 9 | 5.0 | 4 | 2 | 1 |
| Comparative Experimental Example 10 | 9.2 | 4 | 1 | 1 |
| Comparative Experimental Example 11 | 10.3 | 4 | 0 | 0 |

**[Table 32]**

| | pH | Remaining Cryj1 allergen activity | | |
|---|---|---|---|---|
| | | 1 day after | 7 days after | 14 days after |
| Experimental Example 35 | 6.0 | 4 | 3 | 2 |
| Experimental Example 36 | 7.3 | 4 | 3 | 2 |
| Experimental Example 37 | 8.1 | 4 | 3 | 2 |
| Comparative Experimental Examples 12 | 4.0 | 0 | 0 | 0 |
| Comparative Experimental Example 13 | 4.3 | 2 | 0 | 0 |
| Comparative Experimental Example 14 | 8.9 | 4 | 2 | 1 |
| Comparative Experimental Example 15 | 9.8 | 3 | 0 | 0 |

**[Table 33]**

| | pH | Remaining Cryj1 allergen activity | | |
|---|---|---|---|---|
| | | 1 day after | 7 days after | 14 days after |
| Experimental Example 38 | 6.3 | 5 | 5 | 4 |
| Experimental Example 39 | 6.7 | 5 | 5 | 4 |
| Experimental Example 40 | 7.4 | 5 | 5 | 4 |
| Experimental Example 41 | 8.5 | 5 | 4 | 3 |
| Experimental Example 42 | 5.5 | 5 | 4 | 3 |
| Comparative Experimental Example 16 | 4.2 | 5 | 0 | 0 |
| Comparative Experimental Example 17 | 10.0 | 5 | 3 | 2 |

Tables 31 to 33 suggest that because the pH was adjusted in the range of 5.5 to 8.5, the pharmaceutical compositions of Experimental Examples maintained allergen activity scores of 3 or higher even after 14 days from the preparation.
In contrast, the pharmaceutical compositions of Comparative Experimental Examples, the pH of which was out of the range of 5.5 to 8.5, were given poor allergen activity scores, compared to the pharmaceutical compositions of Experimental Examples.

**[Table 34]**

| | pH | Remaining Cryj1 allergen activity | | | |
|---|---|---|---|---|---|
| | | 7 days after | 14 days after | 30 days after | 60 days after |
| Experimental Example 43 | 6.0 | 5 | 5 | 5 | 5 |
| Experimental Example 44 | 6.9 | 5 | 5 | 5 | 5 |
| Experimental Example 45 | 8.2 | 5 | 5 | 5 | 5 |
| Experimental Example 46 | 8.5 | 5 | 5 | 5 | 5 |
| Comparative Experimental Example 18 | 4.4 | 5 | 4 | 4 | 3 |
| Comparative Experimental Example 19 | 5.1 | 5 | 5 | 4 | 4 |
| Comparative Experimental Example 20 | 9.0 | 5 | 5 | 4 | 4 |
| Comparative Experimental Example 21 | 9.9 | 5 | 4 | 4 | 3 |

**[Table 35]**

| | pH | Remaining Cryj1 allergen activity | | | |
|---|---|---|---|---|---|
| | | 7 days after | 14 days after | 30 days after | 60 days after |
| Experimental Example 47 | 5.8 | 5 | 5 | 5 | 5 |
| Experimental Example 48 | 6.7 | 5 | 5 | 5 | 5 |
| Experimental Example 49 | 8.0 | 5 | 5 | 5 | 5 |
| Experimental Example 50 | 8.5 | 5 | 5 | 5 | 5 |
| Comparative Experimental Example 22 | 4.9 | 4 | 3 | 2 | 1 |
| Comparative Experimental Example 23 | 9.0 | 5 | 5 | 4 | 4 |
| Comparative Experimental Example 24 | 10.0 | 5 | 5 | 4 | 3 |

Tables 34 and 35 suggest that because the pH was adjusted in the range of 5.5 to 8.5, all of the jelly preparations of Experimental Examples maintained the best allergen activity score 5 through the test. This indicates that the jelly preparations of Experimental Examples showed more pronounced stabilization effect than the pharmaceutical compositions of Experimental Examples.
In contrast, all the jelly preparations of Comparative Experimental Examples, the pH of which is out of the range of 5.5 to 8.5, were given poor allergen activity scores, compared to the jelly preparations of Experimental Examples.

The pharmaceutical composition of the present invention is an agent that is useful in the prevention or treatment of allergy symptoms and can stably maintain a heat-labile allergen, and therefore is particularly useful in the storage and delivery of such an allergen.

## Claims

1. A pharmaceutical composition comprising:
an allergen, and
at least one selected from the group consisting of an organic acid salt, an inorganic acid salt, and a pH adjuster.

2. The pharmaceutical composition according to claim 1,
wherein the organic acid salt comprises at least one selected from the group consisting of calcium lactate, sodium citrate, calcium citrate, sodium malate, dipotassium glycyrrhizate, disodium glycyrrhizate, calcium gluconate, sodium gluconate, magnesium gluconate, sodium stearyl fumarate, sodium tartrate, potassium sodium tartrate, disodium succinate, sodium acetate, sodium L-aspartate, and sodium L-ascorbate.

3. The pharmaceutical composition according to claim 1 or 2,
wherein the inorganic acid salt comprises at least one selected from the group consisting of calcium carbonate, (anhydrous) calcium hydrogen phosphate, magnesium carbonate, calcium silicate, magnesium silicate, magnesium aluminometasilicate, synthetic aluminum silicate, sodium hydrogen carbonate, sodium hydrogen phosphate, sodium dihydrogen phosphate, potassium hydrogen carbonate, potassium dihydrogen phosphate, and calcium dihydrogen phosphate.

4. The pharmaceutical composition according to any one of claims 1 to 3,
wherein the allergen is a Cryptomeria japonica pollen allergen protein.

5. The pharmaceutical composition according to any one of claims 1 to 4, further comprising gelatin.

6. The pharmaceutical composition according to any one of claims 1 to 5, further comprising water.

7. The pharmaceutical composition according to any one of claims 1 to 6,
wherein the pH adjuster is capable of adjusting the pharmaceutical composition to a pH of 5.5 to 8.5.

8. The pharmaceutical composition according to any one of claims 1 to 7,
wherein the pH adjuster comprises at least one selected from the group consisting of acetic acid, phosphoric acid, boric acid, a mixture of these, sodium hydroxide, and sodium carbonate.

9. The pharmaceutical composition according to any one of claims 1 to 5, 7 and 8,
wherein the pharmaceutical composition does not contain water.

10. The pharmaceutical composition according to any one of claims 1 to 9,
wherein the pharmaceutical composition is a solid preparation, a liquid preparation, or a jelly preparation.

11. The pharmaceutical composition according to any one of claims 1 to 10,
wherein the pharmaceutical composition is for oral administration.

12. The pharmaceutical composition according to any one of claims 1 to 11,
wherein the pharmaceutical composition is for hyposensitization therapy.

13. The pharmaceutical composition according to any one of claims 1 to 10 and 12,
wherein the pharmaceutical composition is for administration by subcutaneous injection.

14. A method for producing a pharmaceutical composition, comprising:
dissolving or dispersing, in water, an allergen and at least one selected from the group consisting of an organic acid salt, an inorganic acid salt, and a pH adjuster, thereby providing an allergen-containing aqueous solution; and
lyophilizing the allergen-containing aqueous solution.

15. The method for producing a pharmaceutical composition according to claim 14,
wherein gelatin is further dissolved in the allergen-containing aqueous solution.

16. The method for producing a pharmaceutical composition according to claim 14 or 15,
wherein the allergen-containing aqueous solution has a pH of 5.5 to 8.5.
